(19) Europäisches Patentamt
European Patent Office
Office européen des brevets

(11) **EP 3 466 386 B1**

(12) **EUROPEAN PATENT SPECIFICATION**

(45) Date of publication and mention
of the grant of the patent:
**20.04.2022 Bulletin 2022/16**

(21) Application number: **17806333.5**

(22) Date of filing: **11.05.2017**

(51) International Patent Classification (IPC):
***A61F 13/511*** (2006.01)   ***A61F 13/49*** (2006.01)

(52) Cooperative Patent Classification (CPC):
**A61F 13/84; A61F 13/51121; A61F 13/51401;**
**A61F 13/515; A61F 13/539;** A61F 2013/51452

(86) International application number:
**PCT/JP2017/017956**

(87) International publication number:
**WO 2017/208775 (07.12.2017 Gazette 2017/49)**

(54) **ABSORBENT ARTICLE**

SAUGFÄHIGER ARTIKEL

ARTICLE ABSORBANT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.05.2016   JP 2016107845**
       **02.05.2017   JP 2017091606**

(43) Date of publication of application:
**10.04.2019 Bulletin 2019/15**

(73) Proprietor: **Kao Corporation**
**Chuo-ku**
**Tokyo 103-8210 (JP)**

(72) Inventors:
• **KOYAMA, Izumi**
  **Haga-gun**
  **Tochigi 321-3497 (JP)**
• **FUKUDA, Yuko**
  **Haga-gun**
  **Tochigi 321-3497 (JP)**

• **KAJIWARA, Jun**
  **Haga-gun**
  **Tochigi 321-3497 (JP)**
• **OKUDA, Yasuyuki**
  **Haga-gun**
  **Tochigi 321-3497 (JP)**

(74) Representative: **Vossius & Partner**
**Patentanwälte Rechtsanwälte mbB**
**Siebertstrasse 3**
**81675 München (DE)**

(56) References cited:
**EP-A1- 1 157 679       WO-A1-91/14414**
**WO-A1-2015/182441    WO-A1-2018/092309**
**JP-A- 2004 298 467    JP-A- 2007 289 561**
**JP-A- 2008 229 246    JP-A- 2010 240 110**
**JP-A- 2013 111 326    JP-A- 2015 107 303**
**JP-A- 2015 142 721    JP-A- 2016 034 341**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to an absorbent article such as a disposable diaper.

Background Art

Conventionally, there is a known disposable diaper whose surface that comes into contact with skin is provided with a sheet or the like for absorbing sweat, thereby effectively preventing eczema, heat rashes, rashes, and the like (JP 2004-298467 A, for example).
JP 2004-298467 A discloses a disposable diaper whose end flaps and side flaps are provided with a composite sheet in which a hydrophobic first fiber nonwoven fabric on the skin-facing surface and a hydrophilic second fiber nonwoven fabric on the non-skin-facing surface are laminated and fixed to each other at fusion bonded portions, in order to absorb sweat. Furthermore, JP 2004-298467 A describes that a large number of fusion bonded portions are formed in the composite sheet, the fusion bonded portions being depressed from the skin-facing side toward the non-skin-facing side.
As other techniques, JP 2002-20957 A and JP H9-31823 A each disclose a layered nonwoven fabric that has a structure in which a hydrophobic fiber layer and a hydrophilic fiber layer are laid one on top of the other in layers and that is used with the hydrophobic fiber layer side being positioned on the skin-facing surface side.
JP 2008 229 246 A discloses another example of an absorbent article.

Summary of Invention

The present invention is directed to an absorbent article including an elasticized region as defined in the claims.

Brief Description of Drawings

[FIG. 1] FIG. 1 is a perspective view of a pull-on disposable diaper that is an embodiment of an absorbent article of the present invention.
[FIG. 2] FIG. 2 is an unfolded plan view of the diaper shown in FIG. 1 in its flat-out, uncontracted state, viewed from the skin-facing surface side.
[FIG. 3] FIG. 3 is a cross-sectional view of a sweat-absorbent and quick-drying sheet taken along line III-III shown in FIG. 2.
[FIG. 4] FIG. 4 is a cross-sectional view taken along line IV-IV shown in FIG. 2.
[FIG. 5] FIG. 5 is an enlarged cross-sectional view showing a cross-section taken along a lateral direction (Y direction) of an outer cover of a waist elasticized portion in the diaper shown in FIG. 1.
[FIG. 6] FIG. 6 is a partial enlarged plan view showing the diaper in its flat-out, uncontracted state, viewed from the non-skin-facing surface side.
[FIG. 7] FIG. 7 is a cross-sectional view corresponding to FIG. 2 and showing a pull-on disposable diaper of an embodiment of the invention.

Description of Embodiments

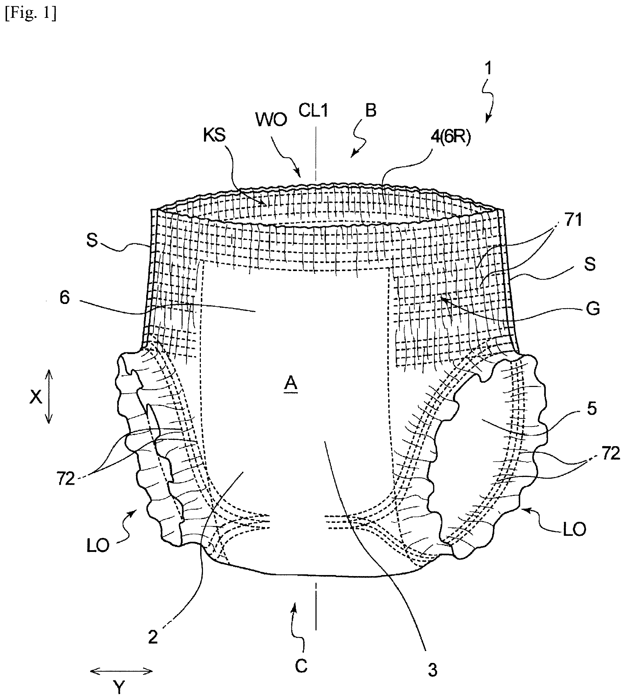

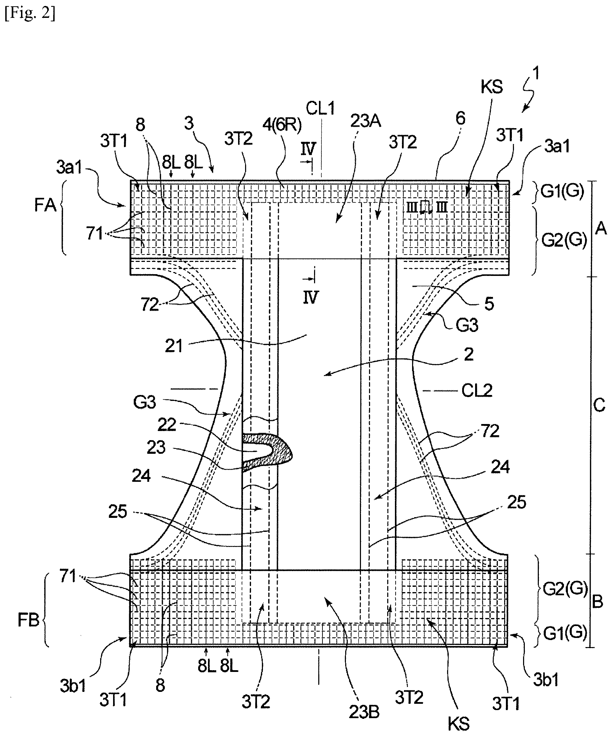

A so-called sweat-absorbent sheet provided in the disposable diaper disclosed in JP 2004-298467 A cannot absorb small drops of sweat that are excreted at the onset of sweating and therefore cannot prevent skin issues such as eczema, heat rashes, and rashes, and thus, there is room for further improvement.
Furthermore, in JP 2002-20957 A and JP H9-31823 A, there is no mention to the effect that the layered nonwoven fabric is disposed in an elasticized region of an absorbent article such as a disposable diaper.
The present invention relates to an absorbent article that can solve the above-mentioned problems in the related art.
Hereinafter, the present invention will be described with reference to the drawings on the basis of preferred embodiments thereof.
An absorbent article of the present invention includes an elasticized region. As shown in FIG. 1, a disposable diaper 1 (hereinafter, alternatively referred to as a "diaper 1") that is a preferred embodiment of the absorbent article of the present invention includes, along a longitudinal direction X, a ventral side region A, a dorsal side region B, and a crotch region C that is positioned between the ventral side region A and the dorsal side region B, has an absorbent member 23 spanning from the ventral side region A to the dorsal side region B, and has a ventral side waist flap FA and a dorsal side waist flap FB extending in a lateral direction Y and positioned outward of front and rear ends 23A and 23B, respectively, in the longitudinal direction X of the absorbent member 23. The ventral side region A is a region that is worn about a ventral side of a wearer when the absorbent article such as a disposable diaper is worn, and the dorsal side region B is a region that is worn about a dorsal side of the wearer when the absorbent article such as a disposable diaper is worn. As shown in FIG. 2, the ventral side waist flap FA refers to a region obtained by adding a region extending in the lateral direction Y and positioned outward in the longitudinal direction X from an edge of the front end 23A on the ventral side region A side in the longitudinal direction X of the absorbent member 23 and regions extending in the lateral direction Y from the front end 23A on the ventral side region A side in the longitudinal direction X of the absorbent member 23. Furthermore, the dorsal side waist flap FB refers to a region obtained by adding a region extending in the lateral direction Y and positioned outward in the longitudinal direction X from an edge of the rear end 23B on the dorsal side region B side in the longitudinal direction X of the absorbent member 23 and regions extending in the lateral direction Y from the rear end 23B on the dorsal side region B side in the longitudinal direction X of the absorbent member 23.

FIGS. 1 and 2 show the diaper 1 that is an embodiment of the absorbent article of the present invention. As shown in FIGS. 1 and 2, the diaper 1 includes an absorbent assembly 2, and an outer cover 3 that is arranged on the non-skin-facing surface side of the absorbent assembly 2 and fixes the absorbent assembly 2. The outer cover 3 has the ventral side waist flap FA and the dorsal side waist flap FB extending in the lateral direction Y and positioned outward of the front and rear ends 23A and 23B, respectively, in the longitudinal direction X of the absorbent member 23 forming the absorbent assembly 2. In the diaper 1, elasticized regions are formed in the ventral side waist flap FA and the dorsal side waist flap FB.

As shown in FIG. 2, the diaper 1 is a so-called pull-on disposable diaper in which both lateral side edges 3al, 3al of the ventral side region A of the outer cover 3 and both lateral side edges 3b1, 3b1 of the dorsal side region B of the outer cover 3 are joined to each other, and form a pair of side seals S, S, a waist opening WO, and a pair of leg openings LO, LO (see FIG. 1). Preferably, in plan view of the diaper 1 in its flat-out, uncontracted state shown in FIG. 2, the outer cover 3 is divided into the ventral side region A that is worn about a ventral side of a wearer when worn, the dorsal side region B that is worn about a dorsal side of the wearer when worn, and the crotch region C that is positioned between the ventral side region A and the dorsal side region B.

The above-described flat-out, uncontracted state of the diaper 1 is, as shown in FIG. 2, a state in which the side seals S are peeled away so that the diaper 1 is in a flat-out state, elastic members at respective portions of the diaper 1 in the flat-out state are uncontracted so that the dimensions of the diaper match its design dimensions (that are the same as the dimensions when the diaper 1 is spread out in a planar shape while completely eliminating the influence of the elastic members).

In this specification, "skin-facing surface" refers to a surface of the diaper 1 or a constituent element thereof (e.g., the absorbent assembly 2) that faces the skin side of the wearer when the diaper 1 is worn, and "non-skin-facing surface" is a surface of the diaper 1 or a constituent element thereof that faces the side (garment side) opposite to the skin side of the wearer when the diaper 1 is worn. Moreover, in the diaper 1, the longitudinal direction (X direction) is a direction from the ventral side region A to the dorsal side region B in plan view of the diaper 1 in its flat-out, uncontracted state. Furthermore, the lateral direction (Y direction) is a direction that is orthogonal to the longitudinal direction (X direction) and is the width direction of the diaper 1 in plan view of the diaper 1 in its flat-out, uncontracted state.

Furthermore, the diaper 1 is bilaterally symmetric about a longitudinal center line CL1 extending in the longitudinal direction (X direction) shown in FIG. 2. A CL2 in FIG. 2 is a lateral center line extending in the lateral direction (Y direction) and dividing the diaper 1 into two halves, and is orthogonal to the longitudinal center line CL1.

In the diaper 1, as shown in FIG. 2, the absorbent assembly 2 has an oblong shape that is relatively oblong in the longitudinal direction (X direction) in the flat-out, uncontracted state of the diaper 1. The absorbent assembly 2 includes a liquid permeable topsheet 21 that forms the skin-facing surface, a sparingly liquid permeable (or water repellent) backsheet 22 that forms the non-skin-facing surface, and the liquid-retentive absorbent member 23 that is positioned between the sheets 21 and 22. Furthermore, as shown in FIG. 2, leak-proof cuffs 24, 24 having elastic members arranged in a state of being stretched in the longitudinal direction (X direction) are provided on both sides along the longitudinal direction (X direction) of the absorbent assembly 2. Specifically, the leak-proof cuffs 24 are made of a liquid impermeable or water repellent and air permeable material, and the leak-proof cuff forming elastic members 25 are arranged in a state of being stretched in the longitudinal direction (X direction), near free ends of the leak-proof cuffs 24. When the diaper is worn, the free ends side of the leak-proof cuffs 24 stand up due to contraction of the leak-proof cuff forming elastic members 25, thereby preventing a bodily fluid from flowing out in the lateral direction (Y direction).

As shown in FIG. 2, the thus configured absorbent assembly 2 is joined to the middle of the outer cover 3 using an assembly fixing adhesive such that the longitudinal direction (X direction) of the absorbent assembly 2 matches the longitudinal direction (X direction) of the diaper 1 in its flat-out, uncontracted state. In this manner, the outer cover 3 is arranged on and adhesively fixed to the non-skin-facing surface side of the backsheet 22 forming the absorbent assembly 2 in the thickness direction of the disposable diaper 1. Accordingly, in the diaper 1, the absorbent member 23 forming the absorbent assembly 2 is arranged spanning from the ventral side region A to the dorsal side region B.

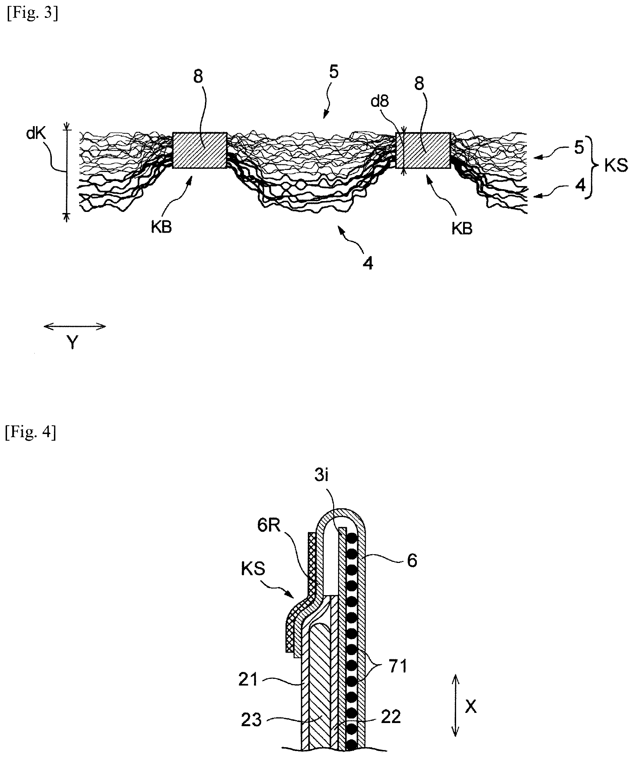

With regard to the absorbent article of the present invention, each of the elasticized regions has a sweat-absorbent and quick-drying sheet that is constituted by at least two layers and disposed on the skin-facing surface side, and it is preferable that at least the dorsal side waist flap FB is provided with a sweat-absorbent and quick-drying sheet. In the diaper 1, as shown in FIG. 2, the dorsal side waist flap FB and the ventral side waist flap FA are respectively provided with sweat-absorbent and quick-drying sheets KS. Each sweat-absorbent and quick-drying sheet KS includes a hydrophobic layer 4 that forms the skin-facing surface thereof, and a hydrophilic layer 5 that is disposed adjacent to a non-skin-facing surface of the hydrophobic layer 4, and has a plurality of fusion-bonded joined regions 8 via which the hydrophobic layer 4 and the hydrophilic layer 5 are fixed to each other. The hydrophobic layer 4 is formed of a hydrophobic fiber and is hydrophobic. The hydrophilic layer 5 is formed of a hydrophilic fiber and is hydrophilic. In the diaper 1, the sweat-absorbent and quick-drying sheets KS are formed by a plurality of sheets being laid one on top of another. Preferably, in the diaper 1, as shown in FIG. 3, the sweat-absorbent and quick-drying sheets KS each have a hydrophobic sheet (hereinafter also referred to as "hydrophobic sheet 4") serving as the hydrophobic layer 4 that forms the skin-facing surface and a hydrophilic sheet (hereinafter also referred to as "hydrophilic sheet 5") serving as the hydrophilic layer 5 adjacent to the non-skin-facing surface of the hydrophobic sheet 4. In the diaper 1, as shown in FIG. 2, the sweat-absorbent and quick-drying sheets KS, which each have the hydrophobic sheet 4 and the hydrophilic sheet 5, are respectively disposed on the skin-facing surface side of the elasticized region in the ventral side waist flap FA and the dorsal side waist flap FB. Furthermore, as shown in FIG. 4, on the non-skin-facing surface side of each sweat-absorbent and quick-drying sheet KS, a folded-back portion 6R of an outer sheet 6 formed of a different sheet than the sweat-absorbent and quick-drying sheet KS, an inner sheet 3i, and the outer sheet 6 are arranged, and the folded-back portion 6R, the inner sheet 3i, and the outer sheet 6 are arranged in this order toward the non-skin-facing surface side.

Preferably, as shown in FIG. 2, in the diaper 1, the outer cover 3 includes the ventral side waist flap FA and the dorsal side waist flap FB extending in the lateral direction Y and positioned outward of the front and rear ends 23A and 23B, respectively, in the longitudinal direction X of the absorbent member 23. As shown in FIGS. 2 and 4, the outer cover 3 includes the outer sheet 6 on the non-skin-facing surface side that forms the outer face of the diaper 1, and the inner sheet 3i disposed on the skin-facing surface side of the outer sheet 6. The outer sheet 6 includes the folded-back portions 6R that are obtained by extension portions that extend from front and rear ends, respectively, of the inner sheet 3i in the longitudinal direction (X direction) being folded back toward the skin-facing surface of the inner sheet 3i. In addition, the hydrophilic sheets 5, which are the hydrophilic layers constituting the respective sweat-absorbent and quick-drying sheets KS, are disposed on the side that is closest to the skin-facing surface of the diaper 1 (the skin-facing surface side of the respective folded-back portions 6R) so as to face the respective folded-back portions 6R. The sweat-absorbent and quick-drying sheets KS are each a composite sheet in which the hydrophobic sheet 4 and the hydrophilic sheet 5 are fixed to each other via the plurality of fusion-bonded joined regions 8.

More preferably, as shown in FIG. 2, in the diaper 1, the sheet material forming the outer sheet 6 in both the ventral side region A and the dorsal side region B is folded back toward the skin-facing surface of the inner sheet 3i along the peripheral edge of the waist opening WO, and the folded-back portions 6R cover the front and rear ends 23A and 23B in the longitudinal direction (X direction) of the absorbent member 23 forming the absorbent assembly 2. The sweat-absorbent and quick-drying sheets KS are further disposed on the skin-facing surface side of the respective folded-back portions 6R. Thus, the outer sheet 6 and the folded-back portions 6R of the outer sheet 6, and the hydrophobic sheet 4 of each sweat-absorbent and quick-drying sheet KS are separate, different sheets. As shown in FIG. 2, in the diaper 1, the outer sheet 6 other than the folded-back portions 6R and the inner sheet 3i are inwardly narrowed toward the longitudinal center line CL1 at the middle in the longitudinal direction (X direction), and are formed so as to have the same shape and the same size. The folded-back portions 6R of the outer sheet 6 are each formed in the shape of a rectangle that is elongated in the lateral direction (Y direction). In the diaper 1, each sweat-absorbent and quick-drying sheet KS that is formed of the hydrophobic sheet 4 and the hydrophilic sheet 5 joined and fixed to each other via the plurality of fusion-bonded joined regions 8 arranged in a dispersed manner through fusion bonding and that has the shape of a rectangle elongated in the lateral direction (Y direction) is laid on the skin-facing surface of a corresponding one of the folded-back portions 6R so that the lateral direction (Y direction) of the sweat-absorbent and quick-drying sheet KS matches the lateral direction (Y direction) of the folded-back portion 6R of the outer sheet 6, and is adhesively bonded to the skin-facing surface of the folded-back portion 6R of the outer sheet 6 with an adhesive. Thus, the dorsal side waist flap FB and the ventral side waist flap FA having the respective sweat-absorbent and quick-drying sheets KS are formed.

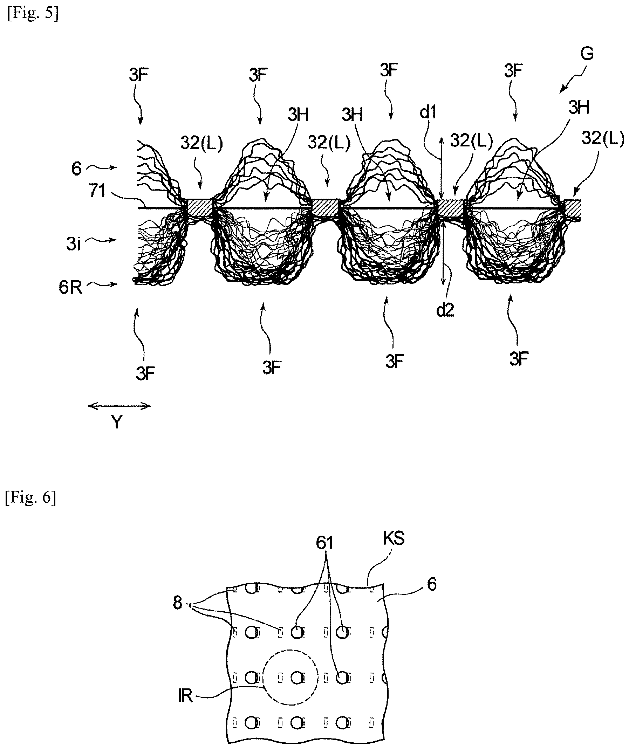

In the diaper 1, as shown in FIGS. 2 and 5, the outer cover 3 has a plurality of elastic members 71 arranged in a state of being stretched in the lateral direction (Y direction) between the outer sheet 6 and the inner sheet 3i and intermittently arranged along the longitudinal direction (X direction). In the diaper 1, waist elasticized portions G1 and below-waist lower elasticized portions G2 are formed by providing the plurality of elastic members 71. Furthermore, in the diaper 1, as shown in FIG. 2, a plurality of leg elastic members 72 in a stretched state are arranged between the outer sheet 6 and the inner sheet 3i. In the diaper 1, leg elasticized portions G3 are formed by providing the plurality of leg elastic members 72. The elasticized regions of the diaper 1 have the waist elasticized portions G1, the below-waist lower elasticized portions G2, and the leg elasticized portions G3.

As shown in FIG. 2, in the diaper 1, the waist elasticized portions G1 are formed in the end flaps that are positioned outward in the longitudinal direction X of the edges of the front and rear ends 23A and 23B in the longitudinal direction (X direction) of the absorbent member 23 forming the absorbent assembly 2 (on the side opposite to the lateral center line CL2), in the longitudinal direction (X direction). Furthermore, in the diaper 1, the below-waist lower elasticized portions G2 are formed in the side flaps that are positioned between lower ends on the lateral center line CL2 side of the waist elasticized portions G1 and lower ends of the side seals S, in the longitudinal direction (X direction). The dorsal side waist flap FB and the ventral side waist flap FA described above are regions obtained by adding the end flaps (the waist elasticized portions G1) and part of the side flaps (the below-waist lower elasticized portions G2). Furthermore, as shown in FIG. 2, in the diaper 1, the leg elasticized portions G3 are formed along the peripheral edges of the leg openings LO.

Furthermore, in the diaper 1, the outer sheet 6 and the inner sheet 3i are provided with outer cover joined regions 32 that are intermittently arranged in a row along the longitudinal direction (X direction) in a region spanning from the waist elasticized portion G1 to the below-waist lower elasticized portion G2 of each of the ventral side region A and the dorsal side region B, and a plurality of fusion-bonded joined region rows L formed of the plurality of outer cover joined regions 32 are arranged at intervals along the lateral direction (Y direction).

The outer cover joined regions 32 are formed by laminating the inner sheet 3i and the outer sheet 6, and melting and solidifying the laminated portions through heat embossing. The heat embossing apparatus may be an apparatus including an embossing roller and an anvil roller, wherein the outer peripheral face of the embossing roller has projections having a shape that conforms to the outer cover joined regions 32. As processing for forming the outer cover joined regions 32, ultrasonic sealing, a laser, and the like may be used instead of heat embossing.

In order to facilitate fitting to the wearer's body and absorption of sweat, in the diaper 1, the interval between cover joined portions 32, 32 that are adjacent to each other in the longitudinal direction (X direction) is preferably 1 mm or greater, and more preferably 3 mm or greater, is preferably 20 mm or less, and more preferably 18 mm or less, and, specifically, is preferably from 1 to 20 mm, and more preferably from 3 to 18 mm.

Furthermore, in order to improve the ability to evaporate sweat that has been absorbed by the sweat-absorbent and quick-drying sheets KS, the interval between pleats 3F, 3F, which will be described later, that are adjacent to each other in the lateral direction (Y direction) is preferably 4 mm or greater, and more preferably 6 mm or greater, is preferably 20 mm or less, and more preferably 16 mm or less, and, specifically, is preferably from 4 to 20 mm, and more preferably from 6 to 16 mm. Note that the interval between pleats 3F that are adjacent to each other in the lateral direction (Y direction) means the interval between the peaks of pleats 3F that are adjacent to each other in the lateral direction (Y direction) in a state in which the elastic members 71 are contracted.

As shown in FIG. 2, in the diaper 1, in a region spanning from the waist elasticized portion G1 to the below-waist lower elasticized portion G2 of each of the ventral side region A and the dorsal side region B, the plurality of elastic members 71 are arranged in a state of being stretched in the lateral direction (Y direction) through gaps between the fusion-bonded joined regions of the plurality of fusion-bonded joined region rows L. In this manner, in the diaper 1, the elastic members 71 are arranged between the outer sheet 6 and the inner sheet 3i that are fixed to each other via the fusion-bonded joined regions, thereby forming the elasticized regions.

Furthermore, as shown in FIG. 2, in the diaper 1, a pair of outer-side fixing regions 3T1, 3T1 formed by joining the outer sheet 6 and the inner sheet 3i using an elastic member fixing adhesive are arranged in both lateral side edges 3a1, 3a1 of the ventral side region A of the outer cover 3 and both lateral side edges 3b1, 3b1 of the dorsal side region B of the outer cover 3, and a pair of assembly-side fixing regions 3T2, 3T2 formed by joining the outer sheet 6 and the inner sheet 3i using an elastic member fixing adhesive are arranged near both sides along the longitudinal direction (X direction) of the absorbent assembly 2.

In each of the waist elasticized portions G1 of the diaper 1, the plurality of elastic members 71 are arranged spanning between the pair of outer-side fixing regions 3T1, 3T1, and the elastic members 71 are fixed between the outer sheet 6 and the inner sheet 3i in both of the pair of outer-side fixing regions 3T1, 3T1, but are not fixed to either the outer sheet 6 or the inner sheet 3i between the pair of outer-side fixing regions 3T1, 3T1.

Furthermore, in each of the below-waist lower elasticized portions G2 of the diaper 1, the plurality of elastic members 71 are arranged spanning from the outer-side fixing region 3T1 to the assembly-side fixing region 3T2, and the elastic members 71 are fixed between the outer sheet 6 and the inner sheet 3i in all the outer-side fixing regions 3T1 and the assembly-side fixing regions 3T2, but are not fixed to either the outer sheet 6 or the inner sheet 3i between the outer-side fixing regions 3T1 and the assembly-side fixing regions 3T2. In each of the below-waist lower elasticized portions G2 of the diaper 1, the elastic members 71 are not arranged between the pair of assembly-side fixing regions 3T2, 3T2, or are arranged therebetween in a state of being finely cut so as not to exert their elasticity.

In each of the leg elasticized portions G3 of the diaper 1, the plurality of leg elastic members 72 arranged in a state of being stretched along the peripheral edges of the leg openings LO are fixed between the outer sheet 6 and the inner sheet 3i via an elastic member fixing adhesive arranged in a planar shape along the peripheral edges of the leg openings LO.

In the outer cover 3 of the diaper 1, as shown in FIG. 4, due to contraction of the plurality of elastic members 71 arranged in a state of being stretched in the lateral direction (Y direction) between the outer sheet 6 and the inner sheet 3i in the waist elasticized portions G1 and the below-waist lower elasticized portions G2, the outer sheet 6 between the fusion-bonded joined region rows L, L that are adjacent to each other in the lateral direction (Y direction) deforms so as to be warped outward, and the inner sheet 3i deforms so as to be warped outward. The pleats 3F formed of the outer sheet 6 or the inner sheet 3i are generated between the fusion-bonded joined region rows L, L that are adjacent to each other, and hollow portions 3H surrounded by the pleats 3F, 3F are formed between the outer sheet 6 and the inner sheet 3i.

In the diaper 1, the hydrophobic sheets 4, which form the respective sweat-absorbent and quick-drying sheets KS, are formed of a hydrophobic nonwoven fabric made of a hydrophobic fiber, and the hydrophilic sheets 5 are formed of a hydrophilic nonwoven fabric made of a hydrophilic fiber. As described above, in the diaper 1, the hydrophobic sheets 4, which form the respective sweat-absorbent and quick-drying sheets KS, are formed of a hydrophobic nonwoven fabric separate from the outer sheet 6. Furthermore, the hydrophilic sheets 5, which form the respective sweat-absorbent and quick-drying sheets KS, are formed of a hydrophilic nonwoven fabric.

The hydrophobic fiber used for the hydrophobic sheets 4 forming the respective sweat-absorbent and quick-drying sheets KS may be a synthetic fiber, and specifically may be those ordinarily used as constituent fibers of various nonwoven fabrics, and examples thereof include: polyolefin fibers such as a polyethylene (PE) fiber and a polypropylene (PP) fiber; fibers using a thermoplastic resin such as polyethylene terephthalate (PET) or polyamide alone; and composite fibers such as core-sheath type and side-by-side type composite fibers.

The hydrophilic fiber used for the hydrophilic sheets 5 forming the respective sweat-absorbent and quick-drying sheets KS may be a fiber obtained by hydrophilizing a synthetic fiber, and specifically may be a fiber obtained by treating a hydrophobic synthetic fiber with a hydrophilizing agent, or a fiber obtained by kneading a hydrophilizing agent into a hydrophobic synthetic fiber during production thereof. Furthermore, a composite nonwoven fabric obtained by mixing a naturally hydrophilic fiber such as a natural or semi-natural fiber with a synthetic fiber also may be used. There is no particular limitation on the synthetic fiber, and examples thereof include: polyolefin fibers such as a polyethylene (PE) fiber and a polypropylene (PP) fiber; fibers using a thermoplastic resin such as polyethylene terephthalate (PET) or polyamide alone; and composite fibers such as core-sheath type and side-by-side type composite fibers. There is no particular limitation on the hydrophilizing agent used to hydrophilize a synthetic fiber, as long as it is an ordinary hydrophilizing agent used for hygiene products. Specific examples of the hydrophilic fiber include: natural cellulose fibers such as wood pulps and non-wood pulps such as a cotton pulp and a straw pulp; and regenerated cellulose fibers such as rayon and cupro.

Whether a sheet is hydrophilic or hydrophobic may be determined based on a contact angle of a constituent fiber measured using "Method for Measuring Contact Angle", which will be described below. Specifically, if the contact angle is 90 degrees or greater, it is determined that the fiber is a hydrophobic fiber with low degrees of hydrophilicity, and, if the contact angle is less than 90 degrees, it is determined that the fiber is a hydrophilic fiber with high degrees of hydrophilicity.

Method for Measuring Fiber Contact Angle

A contact angle is measured, for example, using a contact angle meter MCA-J manufactured by Kyowa Interface Science Co., Ltd. Specifically, immediately after ion exchange water (about 20 picoliters) is dropped onto a surface of a constituent fiber, the contact angle is measured using the contact angle meter. The measurement is performed at five or more different points on the constituent fiber, and an average thereof is calculated as the contact angle. Note that the measurement environment temperature is set to 20°C.

Each sweat-absorbent and quick-drying sheet KS having the hydrophobic sheet 4 and the hydrophilic sheet 5 is a sheet with which, when $1~\mu L \times 49$ drops of a liquid that is assumed as sweat excreted from the skin are uniformly arranged on an acrylic plate and absorbed from the skin-facing surface side of the sheet for 60 seconds, the amount of liquid remaining on the acrylic plate is 10 mg or less, and $50~\mu L \times 1$ drop of the liquid placed on a heat plate that is assumed as the skin is absorbed from the skin-facing surface and evaporates within 15 minutes. There is no particular limitation on the lower limit value of the amount of remaining liquid that is exhibited by the sweat-absorbent and quick-drying sheet KS. The smaller the lower limit value of the amount of remaining liquid, the better, and, and the lower limit value of the amount of remaining liquid is even more preferably 0 mg. The amount of remaining liquid that is exhibited by the sweat-absorbent and quick-drying sheet KS is preferably 5 mg or less, and more preferably 1.7 mg or less. There is no particular limitation on the lower limit value of the evaporation time required for $50~\mu L \times 1$ drop of the liquid placed on the skin-facing surface and absorbed by the sweat-absorbent and quick-drying sheet KS to evaporate, and the shorter the evaporation time, the better. However, if the evaporation time is 5 minutes or longer, a sufficiently satisfactory effect can be obtained. The liquid evaporation time that is exhibited by the sweat-absorbent and quick-drying sheet KS is preferably within 13 minutes, and more preferably within 10 minutes. The amount of remaining liquid and the liquid evaporation time are measured using respective methods below.

Method for Measuring Amount of Remaining Liquid

A sample with dimensions of 5 cm × 5 cm of a sweat-absorbent and quick-drying sheet KS is cut from, for example, the diaper 1, which is an example of the absorbent article. Here, the sweat-absorbent and quick-drying sheet KS and another nonwoven fabric that forms the diaper 1 are distinguished from each other such that a sheet that cannot be separated without breaking up the sheet in portions of the fusion-bonded joined regions 8 is the sweat-absorbent and quick-drying sheet KS, and a sheet that can be separated without breaking up the sheet in portions of the fusion-bonded joined regions 8 is the other nonwoven fabric. Next, pleats and creases in the cut sample of the sweat-absorbent and quick-drying sheet KS are smoothed out so that the entire skin-side surface of the sweat-absorbent and quick-drying sheet KS is flattened. Then, 1 μL of distilled water is dropped onto an acrylic plate with an overall length of 60 mm and a width of 60 mm so that drops of distilled water are arranged in 7 rows × 7 columns at intervals of 7 mm, and the mass (W1) of the acrylic plate in a state in which a total of 49 drops of distilled water are arranged thereon is measured. Subsequently, the cut sample is gently placed on the 49 drops of water arranged on the acrylic plate such that the hydrophobic layer (hydrophobic sheet 4) of the sample faces downward, and is allowed to stand for 60 seconds from the start of dropping. After the lapse of one minute, the sample that has absorbed the distilled water is removed, and the mass (W2) of the acrylic plate at that time is measured. The measurement value is an average when n = 3. Then, the amount of remaining liquid is calculated using an equation below.

$$\text{Amount of remaining liquid (g)} = \text{Mass (W1) of acrylic plate with drops (49 drops) of water arranged thereon} - \text{Mass (W1) of acrylic plate after drops of water have been absorbed by sweat-absorbent and quick-drying sheet KS}$$

It is evaluated that the smaller the amount of remaining liquid (g), the greater the amount of liquid absorbed by the sweat-absorbent and quick-drying sheet KS.

Method for Measuring Liquid Evaporation Time

Measurement is performed using a precision quick thermal property measuring apparatus KES-F7 (Thermo Labo II) manufactured by Kato Tech Co., Ltd. First, a sample with dimensions of 7 cm × 7 cm of a sweat-absorbent and quick-drying sheet KS is cut from, for example, the diaper 1, which is an example of the absorbent article. At this time, pleats and creases are smoothed out so that the entire skin-side surface of the sweat-absorbent and quick-drying sheet KS is flattened. Next, the cut sample is acclimatized under conditions of 23°C and 50% RH for 24 hours. The acclimatized sample is placed on a heat plate (sample table) heated to 33°C in an orientation in which the hydrophobic layer (hydrophobic sheet 4) of the sample faces downward. A wind tunnel is placed on an upper surface of the sample. The sample is exposed to an air stream at a velocity of 0.2 m/second and allowed to stand until the heat supply amount is stabilized, and thereby an initial value is set. Next, 50 μL of distilled water is supplied underneath the sample using a tube preliminarily inserted between the heat plate and the stabilized sample. Changes in the amount of heat that is supplied to the heat plate are continuously measured. The time until the heat supply amount decreases to the initial value is obtained, and the obtained time is used as the liquid evaporation time. Note that the sample continues to be exposed to the air stream at the aforementioned velocity until the liquid evaporation time is obtained. The measurement value is an average when n = 3.

It is evaluated that the shorter the liquid evaporation time, the better the ability to evaporate the liquid that has been absorbed by the sweat-absorbent and quick-drying sheet KS.

Each sweat-absorbent and quick-drying sheet KS, which has the hydrophobic sheet 4 and the hydrophilic sheet 5, is preferably a sheet in which the difference $\Delta q\text{-max}$ (49) between the maximum heat transfer rate of the skin-facing surface of the sheet in a wet state when 1 μL × 49 drops of a liquid have been absorbed within an area of 5 cm × 5 cm of the skin-facing surface for 2 minutes and the maximum heat transfer rate of the skin-facing surface in a humidity-controlled state is preferably 0.15 $kW/m^2$ or less, more preferably 0.13 $kW/m^2$ or less, and even more preferably 0.10 $kW/m^2$ or less. There is no limitation on the lower limit value of the difference $\Delta q\text{-max}$ (49), and the smaller the lower limit value, the better; however, a sufficiently satisfactory effect can be obtained if the difference Aq-max (49) is 0.05 kW/m or greater. The difference Δq-max (49) is measured using a method below.

Method for Measuring Difference Δq-max (49)

Measurement is performed using a precision quick thermal property measuring apparatus KES-F7 (Thermo Labo II) manufactured by Kato Tech Co., Ltd. First, a sample with dimensions of 7 cm × 7 cm of a sweat-absorbent and quick-drying sheet KS is cut from, for example, the diaper 1, which is an example of the absorbent article. At this time, pleats and creases of the sweat-absorbent and quick-drying sheet KS are smoothed out so that the entire skin-side surface of the sweat-absorbent and quick-drying sheet KS is flattened. Next, the cut sample is acclimatized under conditions of 23°C and 50% RH for 24 hours to control the humidity thereof. The sample whose humidity is controlled is placed on a sample table (overall length 205 mm × width 205 mm) made of expanded polystyrene in an orientation in which the hydrophobic layer (hydrophobic sheet 4) of the sample faces upward. With respect to the sample whose humidity is controlled, the maximum heat transfer rate of the hydrophobic layer (hydrophobic sheet 4), which serves as the skin-facing surface, in a humidity-controlled state, that is, the value of qmax (kW/m2) in a humidity-controlled state is measured. At this time, lest the hydrophilic layer (hydrophilic sheet) of the sweat-absorbent and quick-drying sheet KS come into direct contact with the sample table made of expanded polystyrene, the sample is placed on the sample table via another hydrophobic nonwoven fabric spread out thereon. Next, 1 μL of distilled water is dropped onto the hydrophobic layer (hydrophobic sheet 4, skin-facing surface) of the sample with respect to which the value of the qmax (kW/m2) in the humidity-controlled state has been measured. The distilled water is dropped such that drops of distilled water are arranged in 7 rows × 7 columns at intervals of 7 mm. The sample in a state in which a total of 49 drops of distilled water are arranged thereon is allowed to stand, and 2 minutes after dropping of the first drop of distilled water, the maximum heat transfer rate of the hydrophobic layer (hydrophobic sheet 4) in a wet state, that is, the value of qmax (49) (kW/m2) in a wet state is measured. The measurement value is an average when n = 3. Then, the difference Δq-max (49) is calculated using an equation below.

$$\text{Difference } \Delta\text{q-max (49) (kW/m}^2\text{)} = \text{Value of qmax (49) (kW/m}^2\text{) in wet state} -$$

$$\text{Value of qmax (kW/m}^2\text{) in humidity-controlled state}$$

It is evaluated that the smaller the difference Δq-max (49) (kW/m$^2$), the less wet the skin-facing surface of the sweat-absorbent and quick-drying sheet KS, and the better the dry feel.

Each sweat-absorbent and quick-drying sheet KS, which has the hydrophobic sheet 4 and the hydrophilic sheet 5, is preferably a sheet in which the difference Δq-max (245) between the maximum heat transfer rate of the skin-facing surface of the sheet in a wet state in which 5 μL × 49 drops of liquid have been absorbed within an area of 7 cm × 7 cm of the sheet for 2 minutes and the maximum heat transfer rate in a humidity-controlled state is 0.3 kW/m$^2$ or less, more preferably 0.27 kW/m$^2$ or less, and even more preferably 0.25 kW/m$^2$ or less. There is no particular limitation on the lower limit value of the difference Δq-max (245), and the smaller the lower limit value, the better; however, a sufficiently satisfactory effect can be obtained if the lower limit value is 0.15 or greater. The difference Δq-max (245) is measured using a method below.

Method for Measuring Difference Δq-max (245)

Measurement is performed using a precision quick thermal property measuring apparatus KES-F7 (Thermo Labo II) manufactured by Kato Tech Co., Ltd. First, a sample with dimensions of 7 cm × 7 cm of a sweat-absorbent and quick-drying sheet KS is cut from, for example, the diaper 1, which is an example of the absorbent article. At this time, pleats and creases of the sweat-absorbent and quick-drying sheet KS are smoothed out so that the entire skin-side surface of the sweat-absorbent and quick-drying sheet KS is flattened. Next, the cut sample is acclimatized under conditions of 23°C and 50% RH for 24 hours to control the humidity thereof. The sample whose humidity is controlled is placed on a sample table (overall length 205 mm × width 205 mm) made of expanded polystyrene in an orientation in which the hydrophobic layer (hydrophobic sheet 4) of the sample faces upward. With respect to the sample whose humidity is controlled, the maximum heat transfer rate of the hydrophobic layer (hydrophobic sheet 4), which serves as the skin-facing surface, in a humidity-controlled state, that is, the value of qmax (kW/m2) in a humidity-controlled state is measured. At this time, lest the hydrophilic layer (hydrophilic sheet) of the sweat-absorbent and quick-drying sheet KS come into direct contact with the sample table made of expanded polystyrene, the sample is placed on the sample table via another hydrophobic nonwoven fabric spread out thereon. Next, 5 μL of distilled water is dropped onto the hydrophobic layer (hydrophobic sheet 4, skin-facing surface) of the sample with respect to which the value of the qmax (kW/m2) in the humidity-controlled state has been measured. The distilled water is dropped such that drops of distilled water are arranged in 7 rows × 7 columns at intervals of 7 mm. The sample in a state in which a total of 49 drops of distilled water are arranged thereon is allowed to stand, and 2 minutes after dropping of the first drop of distilled water, the maximum heat transfer rate of the hydrophobic layer (hydrophobic sheet 4) in a wet state, that is, the value of qmax (245) (kW/m2) in a wet state is measured. The measurement value is an average when n = 3. Then, the difference $\Delta q$-max (245) is calculated using an equation below.

$$\text{Difference } \Delta q\text{-max (245) (kW/m}^2\text{)} = \text{Value of qmax (245) (kW/m}^2\text{) in wet state} -$$

$$\text{Value of qmax (kW/m}^2\text{) in humidity-controlled state}$$

Similar to the difference $\Delta q$-max (49) (kW/m$^2$), it is evaluated that the smaller the difference $\Delta q$-max (245) (kW/m$^2$), the less wet the skin-facing surface of the sweat-absorbent and quick-drying sheet KS, and the better the dry feel.

Hereinafter, materials for forming the constituent elements of the diaper 1 will be described.

As the topsheet 21, the backsheet 22, the absorbent member 23, the leak-proof cuffs 24, and the like forming the absorbent assembly 2, various types of members conventionally used for absorbent articles such as disposable diapers can be used without any specific limitation. For example, the topsheet 21 may be a monolayered or multilayered nonwoven fabric, an apertured film, or the like. The backsheet 22 may be a moisture permeable resin film or the like. The absorbent member 23 may be an absorbent core made of absorbent polymer particles and fiber materials wrapped in tissue paper. Furthermore, the leak-proof cuffs 24 may be a water repellent monolayered or multilayered nonwoven fabric, or the like.

Examples of the material for forming the elastic members (the leak-proof cuff forming elastic members 25, the elastic members 71, the leg elastic members 72, etc.) include a synthetic rubber, such as styrene-butadiene, butadiene, isoprene, and neoprene, natural rubber, EVA, elastic polyolefin, and polyurethane. With respect to the form of the elastic members, filamentous elastic members (rubber thread, etc.) or string-like elastic members (flat rubber string, etc.) having a rectangular, square, circular, elliptical, or polygonal cross-sectional shape or the like can be preferably used.

As an adhesive 9, the elastic member fixing adhesive for fixing the elastic members (the leak-proof cuff forming elastic members 25, the elastic members 71, the leg elastic members 72, etc.), and the assembly fixing adhesive for fixing the outer cover 3, the absorbent assembly 2, and the like, various types of hot melt adhesives and the like conventionally used for absorbent articles such as disposable diapers can be used without any specific limitation.

Hereinafter, effects that are obtained when the above-described diaper 1 of the embodiment of the present invention is used will be described.

As shown in FIGS. 2 and 3, the diaper 1 includes the sweat-absorbent and quick-drying sheet KS on the skin-facing surface side of the dorsal side waist flap FB, which constitutes the elasticized region. The sweat-absorbent and quick-drying sheet KS includes the hydrophobic layer 4 forming the skin-facing surface and the hydrophilic layer 5 disposed adjacent to the non-skin-facing surface of the hydrophobic layer 4. The sweat-absorbent and quick-drying sheet KS is a sheet in which, when a liquid is absorbed from the hydrophobic layer (hydrophobic sheet 4), which forms the skin-facing surface, the above-described amount of remaining liquid is 10 mg or less, and the evaporation time of a liquid absorbed from the hydrophobic layer (hydrophobic sheet 4), which forms the skin-facing surface, is within 15 minutes. The diaper 1 provided with this sweat-absorbent and quick-drying sheet KS is capable of both absorbing sweat and quickly drying after absorbing sweat, can absorb small drops of sweat excreted at the onset of sweating, can improve a dry tactile feel, and can be expected to reduce skin issues such as eczema, heat rashes, and rashes. Moreover, as shown in FIG. 2, the diaper 1 is provided with the sweat-absorbent and quick-drying sheets KS on the skin-facing surface side of the dorsal side waist flap FB and the ventral side waist flap FA, respectively, and thus, the above-described effects can be more reliably achieved.

Furthermore, each sweat-absorbent and quick-drying sheet KS included in the diaper 1 is a sheet in which the difference $\Delta q$-max (49) is 0.15 kW/m$^2$ or less, and therefore can, in particular, absorb even small drops of sweat excreted at the onset of sweating. Thus, the reduction of skin issues such as eczema, heat rashes, and rashes can be expected even more.

Furthermore, each sweat-absorbent and quick-drying sheet KS included in the diaper 1 is a sheet in which the difference $\Delta q$-max (245) is 0.3 kW/m$^2$ or less, and therefore can, in particular, absorb even large drops of sweat. Thus, the reduction of skin issues such as eczema, heat rashes, and rashes can be expected even more.

Furthermore, as shown in FIG. 2, in the diaper 1, the plurality of elastic members 71 are arranged in a state of being stretched in the lateral direction (Y direction) through gaps between the fusion-bonded joined regions 8 of a plurality of fusion-bonded joined region rows 8L, in a region spanning from the waist elasticized portion G1 to the below-waist lower elasticized portion G2 in each of the ventral side region A and the dorsal side region B. As shown in FIG. 5, when the elastic members 71 arranged in a state of being stretched are contracted, the pleats 3F formed of the outer sheet 6 or the inner sheet 3i are generated between the fusion-bonded joined region rows L, L that are adjacent to each other, and the hollow portions 3H surrounded by the pleats 3F are formed between the outer sheet 6 and the inner sheet 3i. Accordingly, sweat that has been absorbed by the sweat-absorbent and quick-drying sheets more easily evaporate due to the hollow portions 3H, and thus, skin issues such as eczema, heat rashes, and rashes can be further reduced.

Furthermore, in order to facilitate the above-described effects, it is preferable that, in plan view of each sweat-absorbent and quick-drying sheet KS of the diaper 1 in its flat-out, uncontracted state, as shown in FIG. 6, the fusion-bonded joined regions 8 are dispersedly arranged over the entire hydrophobic layer (hydrophobic sheet 4) such that at least one fusion-bonded joined region 8 is partially or entirely contained in an imaginary circle IR with a radius of 3 mm. It is more preferable that the fusion-bonded joined regions 8 are dispersedly arranged over the entire hydrophobic layer (hydrophobic sheet 4) such that at least one fusion-bonded joined region 8 is partially or entirely contained in an imaginary circle IR with a radius of 2 mm. It is even more preferable that the fusion-bonded joined regions 8 are dispersedly arranged over the entire hydrophobic layer (hydrophobic sheet 4) such that at least one fusion-bonded joined region 8 is partially or entirely contained in an imaginary circle IR with a radius of 1.5 mm. Here, the wording "at least one fusion-bonded joined region 8 is partially or entirely contained in an imaginary circle IR" means that, if ten imaginary circles IR are set on the sweat-absorbent and quick-drying sheet KS, one or two of the ten imaginary circles IR may contain no fusion-bonded joined region 8, and it is sufficient that each of the other eight imaginary circles IR partially or entirely contains at least one fusion-bonded joined region 8. The above-described imaginary circles IR assume sweat-secreting portions (sweat glands) that are dispersedly located on the surface of the skin of the human body, and the sweat-absorbent and quick-drying sheet KS is capable of more efficiently absorbing sweat as a result of having the above-described configuration. In particular, an arrangement of the fusion-bonded joined regions 8 that satisfies the above-described configuration when the radius of the imaginary circles IR is 2 mm is more effective, and an arrangement of the fusion-bonded portion joined regions 8 that satisfies the above-described configuration even when the radius of the imaginary circles IR is 1.5 mm is even more effective.

Furthermore, in order to facilitate the above-described effects, it is preferable that, in the hydrophilic layer (hydrophilic sheet 5) forming each sweat-absorbent and quick-drying sheet KS, an orientation direction of the constituent fiber forming the hydrophilic layer matches the stretching and contracting direction of the elasticized region. Here, the wording "the orientation direction of the constituent fiber matches the stretching and contracting direction of the elasticized region" means not only a state in which the orientation direction and the stretching and contracting direction perfectly match the lateral direction (Y direction), for example, but also includes a state in which the orientation direction and the stretching and contracting direction substantially match the lateral direction (Y direction), for example. Since the diaper 1 is contracted due to contraction of the elastic members 71 arranged in a state of being stretched in the lateral direction (Y direction), the stretching and contracting direction of the diaper 1 is the lateral direction (Y direction), and therefore, it is preferable that the orientation direction of the constituent fiber forming the hydrophilic layer is the lateral direction (Y direction). The reason for this is that, if the orientation direction of the constituent fiber forming the hydrophilic layer (hydrophilic sheet 5) is the lateral direction (Y direction), sweat that has been absorbed easily spreads in the lateral direction (Y direction) of the pleats 3F extending in the longitudinal direction (X direction) as a result of the pleats 3F and the hollow portions 3H being formed due to contraction of the elastic members 71 arranged in a stretched state, and thus, the sweat easily evaporates.

Method for Determining Orientation Direction of Constituent Fiber

A target fiber sheet (sweat-absorbent and quick-drying sheet, etc.) obtained from an absorbent article is allowed to stand in an environment at a room temperature of 23 °C $\pm$ 2°C and a relative humidity of 50% RH $\pm$ 2% for 12 hours to thereby control the humidity thereof to a constant state. A measurement sample is cut from the sheet after the humidity is controlled, the measurement sample having a shape with dimensions of 150 mm in the lateral direction (Y direction) of the absorbent article and 25 mm in the longitudinal direction (X direction). This sample is attached to chucks of a tensile testing machine (Autograph AG-X 1 kN manufactured by Shimadzu Corporation) under no tension such that the width direction (Y direction) of the sample matches the pulling direction. The distance between the chucks is set at 100 mm. The sample is pulled at a pulling rate of 300 mm/minute, and the maximum strength is measured until the sample fractures. The measurement is performed 5 times, and the average of the measured values is used as the tensile strength in the width direction (X direction). Furthermore, with regard to the tensile strength in the longitudinal direction (X direction), a sample having a rectangular shape with dimensions of 150 mm in the longitudinal direction (X direction) and 25 mm in the width direction (Y direction) is cut from the sheet after the humidity is controlled, this sample is attached to chucks of a tensile testing machine under no tension such that the longitudinal direction (X direction) of the sample matches the pulling direction, and the tensile strength in the longitudinal direction (X direction) is obtained in the same manner as described above. The direction (lateral direction or longitudinal direction) in which the obtained tensile strength is the greater is determined as the orientation direction of the constituent fiber forming the fiber sheet.

Furthermore, in order to facilitate the above-described effects, with regard to a sweat-absorbent and quick-drying sheet KS formed of a plurality of sheets (hydrophobic sheet 4 and hydrophilic sheet 5) laid one on top of another like the sweat-absorbent and quick-drying sheets KS of the diaper 1, it is preferable that constituent fiber forming the hydrophobic sheet 4, which is the hydrophobic layer, differs from the constituent fiber forming the hydrophilic sheet 5, which is the hydrophilic layer, at least in the degrees of hydrophilicity, of the degrees of hydrophilicity, the fineness, the fiber density, and the constituent resin. In particular, with regard to the difference in degrees of hydrophilicity, specifically, the difference is preferably 10 degrees or greater, and more preferably 20 degrees or greater, in terms of the difference in contact angle of the constituent fibers as measured in accordance with the method described in "Method for Measuring Contact Angle" above.

Furthermore, in order to facilitate the above-described effects and enable absorption of even small drops of sweat excreted at the onset of sweating, in the case where a sweat-absorbent and quick-drying sheet KS is formed of a plurality of sheets (hydrophobic sheet 4 and hydrophilic sheet 5) laid one on top of another as in the diaper 1, the ratio of the entire area of the fusion-bonded joined regions 8 to the entire area of the sweat-absorbent and quick-drying sheet KS is preferably 12% or less, and more preferably 10% or less, is preferably 3% or greater, and more preferably 5% or greater, and, specifically, is preferably from 3 to 12%, and more preferably from 5 to 10%.

Furthermore, in order to facilitate the above-described effects and enable absorption of even small drops of sweat excreted at the onset of sweating, in the case where a sweat-absorbent and quick-drying sheet KS is formed of a plurality of sheets (hydrophobic sheet 4 and hydrophilic sheet 5) laid one on top of another as in the diaper 1, the area of each fusion-bonded joined region 8 is preferably 2 mm$^2$ or less, and more preferably 1.8 mm$^2$ or less, is preferably 0.3 mm$^2$ or greater, and more preferably 0.5 mm$^2$ or greater, and, specifically, is preferably from 0.3 to 2 mm$^2$, and more preferably from 0.5 to 1.8 mm$^2$.

Furthermore, in order to facilitate the above-described effects and facilitate diffusion of sweat that has been absorbed, in the case where a sweat-absorbent and quick-drying sheet KS is formed of a plurality of sheets (hydrophobic sheet 4 and hydrophilic sheet 5) laid one on top of another as in the diaper 1, the basis weight of the entire sweat-absorbent and quick-drying sheet KS is more preferably 40 g/m$^2$ or less, and more preferably 35 g/m$^2$ or less, is preferably 15 g/m$^2$ or greater, and more preferably 20 g/m$^2$ or greater, and, specifically, is preferably from 15 to 40 g/m$^2$, and more preferably from 20 to 35 g/m$^2$.

Furthermore, in the case where a sweat-absorbent and quick-drying sheet KS is formed of a plurality of sheets (hydrophobic sheet 4 and hydrophilic sheet 5) laid one on top of another as in the diaper 1, the basis weight of the hydrophilic sheet 5 forming the sweat-absorbent and quick-drying sheet KS is preferably 9 g/m$^2$ or greater, and preferably 13 g/m$^2$ or greater, is preferably 20 g/m$^2$ or less, and preferably 18 g/m$^2$ or less, and, specifically, is preferably from 9 to 20 g/m$^2$, and preferably from 13 to 18 g/m$^2$.

Furthermore, in order to facilitate the above-described effects and facilitate diffusion of sweat that has been absorbed, in the case where a sweat-absorbent and quick-drying sheet KS is formed of a plurality of sheets (hydrophobic sheet 4 and hydrophilic sheet 5) laid one on top of another as in the diaper 1, the inter-fiber distance of the constituent fiber forming the hydrophilic sheet 5 of the sweat-absorbent and quick-drying sheet KS is preferably shorter than the inter-fiber distance of the fiber forming the hydrophobic sheet 4. The inter-fiber distance of the constituent fiber forming the hydrophilic sheet 5 is preferably 45 $\mu$m or less, and more preferably 35 $\mu$m or less, is preferably 5 $\mu$m or greater, and more preferably 10 $\mu$m or greater, and, specifically, is preferably from 5 to 45 $\mu$m, and more preferably from 10 to 35 $\mu$m.

The above-described inter-fiber distance is measured using a method below.

Method for Measuring Inter-Fiber Distance

The inter-fiber distance can be obtained using Formula 1 below. Formula 1

$$\text{Inter-fiber distance} (\mu m) = 10^4 \sqrt{\frac{10L}{9w} \cdot \left[\frac{1}{\sum_{i=1}^{p} \frac{\alpha i}{D i}}\right]} \quad \cdots \quad (1)$$

where L is the thickness (cm) of a sheet (the hydrophobic sheet 4, or the hydrophilic sheet 5), w is the basis weight (g/m$^2$) of the sheet,
Di is the fiber denier of a constituent fiber i forming the sheet, and
$\alpha$i is the weight proportion (%) of the constituent fiber i.

Furthermore, in order to facilitate the above-described effects, and facilitate diffusion of sweat that has been absorbed, in the case where a sweat-absorbent and quick-drying sheet KS is formed of a plurality of sheets (hydrophobic sheet 4 and hydrophilic sheet 5) laid one on top of another as in the diaper 1, the hydrophilic sheet 5, which is the hydrophilic layer, of the sweat-absorbent and quick-drying sheet KS is preferably a spunbonded nonwoven fabric or a meltblown nonwoven fabric, and more preferably a spunbonded nonwoven fabric.

Furthermore, in order to facilitate the above-described effects and achieve both the sweat absorbing properties and a smooth, dry feel, in the case where a sweat-absorbent and quick-drying sheet KS is formed of a plurality of sheets (hydrophobic sheet 4 and hydrophilic sheet 5) laid one on top of another as in the diaper 1, the basis weight of the hydrophobic sheet 4 of the sweat-absorbent and quick-drying sheet KS is preferably 9 g/m² or greater, and preferably 10 g/m² or greater, is preferably 20 g/m² or less, and preferably 18 g/m² or less, and, specifically, is preferably from 9 to 20 g/m², and preferably from 10 to 18 g/m².

As shown in FIG. 3, each sweat-absorbent and quick-drying sheet KS is formed of the hydrophobic sheet 4 and the hydrophilic sheet 5 laid one on top of the other. As shown in FIG. 3, the sweat-absorbent and quick-drying sheet KS includes depressed portions KB depressed in the thickness direction from the hydrophobic sheet 4 side toward the hydrophilic sheet 5 side, and the fusion-bonded joined regions 8 are provided in the respective depressed portions KB. Each depressed portion KB has a thickness that becomes smaller toward the fusion-bonded joined region 8. The state in which the thickness of each depressed portion KB becomes smaller toward the fusion-bonded joined region 8 has a meaning that encompasses a state in which the thickness of an outer periphery of the depressed portion KB continuously becomes smaller toward the fusion-bonded joined region 8 and a state in which the thickness of the outer periphery of the depressed portion KB becomes smaller toward the fusion-bonded joined region 8 in a stepwise manner. Preferably, the outer periphery of each depressed portion KB has a thickness that gradually becomes smaller toward the fusion-bonded joined region 8. The outer periphery as used herein refers to a portion of the depressed portion KB that extends outward from an outer peripheral wall of the fusion-bonded joined region 8 that is the peripheral edge (contour) of the fusion-bonded joined region 8. The outer periphery is formed in the shape of a ring starting from the outer peripheral wall of the fusion-bonded joined region 8 in each depressed portion KB.

Furthermore, with regard to each sweat-absorbent and quick-drying sheet KS, in order to shift the wearer's sweat toward the hydrophilic sheet 5, the fusion-bonded joined regions 8 in the respective depressed portions KB have a thickness d8 (see FIG. 3) that is preferably 1/3 or less, more preferably 1/4 or less, and even more preferably 1/5 or less of a thickness dK (see FIG. 3) of portions of the sweat-absorbent and quick-drying sheet KS other than the depressed portions KB, and the lower limit of the thickness d8 is 1/30 of the thickness dK.

Specifically, from the same point of view, the thickness d8 (see FIG. 3) of the fusion-bonded joined regions 8 is preferably 60 μm or less, and more preferably 45 μm or less, and the lower limit thereof is 10 μm.

The thickness d8 of the fusion-bonded joined regions 8 is measured as follows. A portion including a fusion-bonded joined region 8 is cut in the thickness direction using a knife, a cutter, a razor, or the like such that the cut face of the fusion-bonded joined region 8 does not collapse, the cut cross-section is observed using a microscope (Digital Microscope VHX-1000 manufactured by Keyence Corporation, for example), and the thickness of the thickest portion is measured. The thickness is measured at ten different fusion-bonded joined regions 8, and an average thereof is calculated as the thickness of the fusion-bonded joined regions 8.

Furthermore, from the same point of view, the thickness dK (see FIG. 3) of the portions of the sweat-absorbent and quick-drying sheet KS other than the depressed portions KB is preferably 600 μm or less, and more preferably 350 μm or less, and the lower limit thereof is 100 μm.

The thickness dK of the portions of the sweat-absorbent and quick-drying sheet KS other than the depressed portions KB forming the sweat-absorbent and quick-drying sheet KS is measured using a thickness gauge in a state in which a load of 0.05 kPa is applied to the sweat-absorbent and quick-drying sheet KS. A laser displacement sensor manufactured by Keyence Corporation (LK-080 manufactured by Keyence Corporation) is used as the thickness gauge. The thickness is measured at ten points, and an average thereof is calculated as the thickness of the portions of the sweat-absorbent and quick-drying sheet KS other than the depressed portions KB.

Furthermore, in order to improve the smooth, dry tactile feel even more, it is preferable that each sweat-absorbent and quick-drying sheet KS has a plurality of through holes passing through the sweat-absorbent and quick-drying sheet KS. The through holes passing through the sweat-absorbent and quick-drying sheet KS may be formed at center portions of the respective fusion-bonded joined regions 8 or may be formed at portions other than the fusion-bonded joined regions 8. In the case where the through holes are formed at the center portions of the respective fusion-bonded joined regions 8, if ultrasonic sealing, a laser, or the like is used to form the fusion-bonded joined regions 8, the through holes can be formed by increasing the output or the melting time compared with those in the case of forming the fusion-bonded joined regions 8. In the case where the through holes are formed at portions other than the fusion-bonded joined regions 8, the through holes can be formed using a pin embossing apparatus or the like. The area of each through hole is preferably from 0.5 to 5 mm², and more preferably from 0.75 to 4 2 mm².

Furthermore, in order to facilitate the above-described effects and make it even easier for sweat that has been absorbed to evaporate, each elasticized region has the outer sheet 6 separate from the sweat-absorbent and quick-drying sheet KS on the side closer to the non-skin-facing surface than the sweat-absorbent and quick-drying sheet KS, and it is preferable that, as shown in FIG. 6, the outer sheet 6 has a plurality of openings 61 passing through the outer sheet 6. The area of each opening 61 is preferably larger than the area of each fusion-bonded joined region 8, and is preferably from 1 to 5 mm$^2$, and more preferably from 1.5 to 4 mm$^2$. As shown in FIG. 6, the openings 61 are preferably arranged such that, in the diaper 1 in its flat-out, uncontracted state, at least some of the openings 61 do not overlap the fusion-bonded joined regions 8 of the sweat-absorbent and quick-drying sheet KS. The reason for this is that if the openings 61 are arranged without overlapping the fusion-bonded joined regions 8 of the sweat-absorbent and quick-drying sheet KS, sweat that has been absorbed by the sweat-absorbent and quick-drying sheet KS and retained and diffused in the hydrophilic layer except for the fusion-bonded joined regions 8 easily evaporates via the openings 61 passing through the outer sheet 6.

Furthermore, in order to facilitate the above-described effects and make it even easier for sweat that has been absorbed to evaporate, in the waist elasticized portion G1 and the below-waist lower elasticized portion G2 of each elasticized region, as shown in FIG. 5, the pleats 3F formed of the outer sheet 6 or the inner sheet 3i are generated, the hollow portions 3H surrounded by the pleats 3F are formed between the outer sheet 6 and the inner sheet 3i, and a gather portion G is provided in which projections formed by the outer sheet 6 being warped toward the non-skin side and depressions formed by the outer sheet 6 being depressed toward the skin side are alternately arranged in the width direction (Y direction) of the diaper 1. When viewed in a cross-section of the gather portion G in a state in which the thus formed elasticized region is stretched by 70%, it is preferable that, as shown in FIG. 5, the openings 61 are arranged in regions of the outer sheet 6 spanning from a position at half a depth d1 of the depressions depressed toward the skin side to peaks of the respective projections warped toward the non-skin side. Preferably, 60% or greater, and more preferably 75% or greater of all of the openings 61 are arranged in such regions, and the upper limit of the percentage of openings 61 arranged in such regions is preferably 100%.

Furthermore, in order to facilitate the above-described effects and make it even easier for sweat that has been absorbed to evaporate, when the gather portion G that is formed due to contraction of the elastic members 71 is viewed in cross section as shown in FIG. 4, preferably 60% or greater, and more preferably 75% or greater of all of the fusion-bonded joined regions 8 in the sweat-absorbent and quick-drying sheet KS are arranged in regions of the hydrophobic sheet 4 spanning from a position at half a depth d2 of depressions depressed toward the non-skin side to peaks of projections (the skin-facing surface side) warped toward the skin side, and the upper limit of the percentage of fusion-bonded joined regions 8 arranged in such regions is preferably 100%.

Although the present invention has been described based on a preferred embodiment above, the present invention is not limited to the foregoing embodiment, and changes can be made thereto as appropriate.

For example (not being claimed), in the above-described diaper 1, the hydrophobic sheets 4 forming the sweat-absorbent and quick-drying sheets KS are bonded to the skin-facing surface side of the respective folded-back portions 6R of the outer sheet 6 via the adhesive as shown in FIG. 2; however, the folded-back portions 6R of the outer sheet 6 may be formed as hydrophobic sheets. Furthermore, although the sweat-absorbent and quick-drying sheets KS of the diaper 1 each have the two sheets, namely, the hydrophobic sheet 4 and the hydrophilic sheet 5, a configuration may also be adopted in which each sweat-absorbent and quick-drying sheet KS further has another sheet on the non-skin-facing surface side of the hydrophilic sheet 5 and is formed by joining three or more sheets including this sheet, the hydrophobic sheet 4, and the hydrophilic sheet 5 together via fusion-bonded joined regions 8 through fusion-bonding. Furthermore, although the sweat-absorbent and quick-drying sheets KS of the diaper 1 are each formed of a plurality of sheets (hydrophobic sheet 4 and hydrophilic sheet 5) laid one on top of another, each sweat-absorbent and quick-drying sheet KS may be formed of a single sheet in which at least the hydrophobic layer 4 and the hydrophilic layer 5 are laid one on top of another. In the case of a sweat-absorbent and quick-drying sheet KS formed of a single sheet of multilayered nonwoven fabric, the multilayered fabric may be a double-layered nonwoven fabric having the two layers, that is, the hydrophobic layer 4 and the hydrophilic layer 5, or may further have another layer on the non-skin-facing surface side of the hydrophilic layer 5.

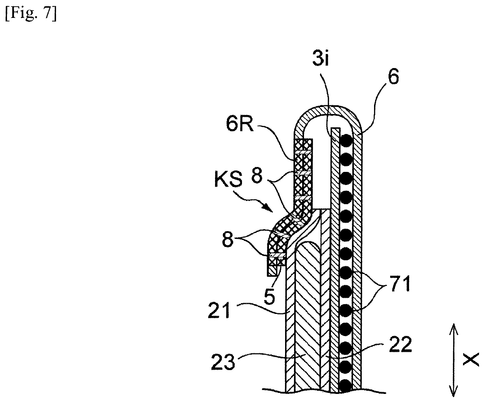

Furthermore, as shown in FIG. 7, a configuration is adopted in which the outer cover 3 has an outer sheet 6 and a hydrophilic sheet 5 that is separate from the outer sheet 6, the outer sheet 6 includes a folded-back portion 6R that is formed by the outer sheet 6 being folded back so as to cover the skin-facing surface side of the hydrophilic sheet 5, and a sweat-absorbent and quick-drying sheet KS is formed of the folded-back portion 6R of the outer sheet 6 and the hydrophilic sheet 5 laid one on top of the other. More specifically, a configuration may be adopted in which the outer sheet 6 is folded back toward the skin-facing surface of the hydrophilic sheet 5 along the peripheral edge of the waist opening WO, the thus formed folded-back portion 6R serves as a hydrophobic sheet 4 that is positioned on the side closest to the skin-facing surface of the diaper 1, and this folded-back portion 6R and the hydrophilic sheet 5 having the shape of a rectangle elongated in the lateral direction (Y direction) are fixed to each other via fusion-bonded joined regions 8, thereby forming a sweat-absorbent and quick-drying sheet KS. In this case, the hydrophilic sheet 5 may be a hydrophilic sheet separate from the hydrophilic inner sheet 3i or may be the hydrophilic inner sheet 3i.

In the diaper 1, as shown in FIGS. 3 and 4, the sweat-absorbent and quick-drying sheets KS are formed by joining a hydrophobic nonwoven fabric made of a hydrophobic fiber and a hydrophilic nonwoven fabric made of a hydrophilic fiber to each other via the fusion-bonded joined regions 8. However, a nonwoven fabric produced by accumulating a hydrophobic fiber and a hydrophilic fiber in layers and then melting and fixing the layers together can also be used as a sweat-absorbent and quick-drying sheet KS. The thus configured sweat-absorbent and quick-drying sheet KS is formed as a single sheet of nonwoven fabric, in which the hydrophobic layer formed of the hydrophobic fiber constitutes the skin-facing surface, and the hydrophilic layer formed of the hydrophilic fiber constitutes the non-skin-facing surface, and has fusion-bonded joined regions 8 for fixing the constituent fiber of the hydrophobic layer and the constituent fiber of the hydrophilic layer to each other. The fusion-bonded joined regions 8 in the case where each sweat-absorbent and quick-drying sheet KS is formed of a single sheet of nonwoven fabric are the same as the fusion-bonded joined regions 8 of the sweat-absorbent and quick-drying sheets KS of the diaper 1, via which the hydrophobic sheet 4 and the hydrophilic sheet 5 are joined to each other, in that a hydrophobic layer formed of a hydrophobic fiber and a hydrophilic layer formed of a hydrophilic fiber are fixed to each other via a plurality of fusion-bonded joined regions, but are different therefrom in that the fusion-bonded joined regions 8 in the case where each sweat-absorbent and quick-drying sheet KS is formed of a single sheet of nonwoven fabric are formed during production of the nonwoven fabric. The fusion-bonded joined regions 8 are formed in order to fix the constituent fiber of the hydrophobic layer and the constituent fiber of the hydrophilic layer to each other and enable the resulting sweat-absorbent and quick-drying sheet KS to maintain the form of a single sheet of nonwoven fabric. Basically, it does not matter whether the fusion-bonded joined regions 8 are arranged in a uniformly dispersed manner or arranged in a non-uniformly dispersed manner as long as the form of a nonwoven fabric can be maintained.

With respect to the method for producing a sweat-absorbent and quick-drying sheet KS formed of a single sheet of nonwoven fabric, various methods with which a nonwoven fabric can be shaped can be used without any specific limitation, but spunbonding is preferable in light of its excellent productivity.

For example, a spunbonded nonwoven fabric is produced in the following manner. A polymer for forming a hydrophobic fiber and a polymer for forming a hydrophilic fiber are separately melted in respective extruders or the like, and the molten products are discharged one after another from respective spinnerets having spinning nozzles to spin and arrange filaments of the hydrophobic fiber and filaments of the hydrophilic fiber in layers. The spun composite filaments are cooled with a cooling fluid, further tensioned with drawing air to a predetermined fineness, and collected and accumulated onto a collecting belt as-is to a predetermined thickness. Then, the accumulated filaments are subjected to heat embossing, and thus fusion-bonded joined regions 8 via which the constituent fiber of the hydrophobic layer and the hydrophilic constituent fiber are joined to each other are formed. In this manner, a sweat-absorbent and quick-drying sheet KS formed of a spunbonded nonwoven fabric can be obtained.

Furthermore, a sweat-absorbent and quick-drying sheet KS formed of a single sheet of nonwoven fabric can also be produced using a method in which a nonwoven fabric formed by laying a carded web mainly composed of a hydrophobic, fusion-bondable fiber and a carded web mainly composed of a hydrophilic fiber one on top of the other and then combining the entire carded webs together through hot air treatment is used as the nonwoven fabric, and the nonwoven fabric is bonded under heat and pressure through heat embossing.

The heat embossing apparatus may be an apparatus including an embossing roller and an anvil roller, wherein the outer peripheral face of the embossing roller has projections having a shape that conforms to the fusion-bonded joined regions 8. As processing for forming the fusion-bonded joined regions 8, ultrasonic sealing, a laser, and the like may be used instead of heat embossing.

In the case where each sweat-absorbent and quick-drying sheet KS is formed of a single sheet of multilayered nonwoven fabric, it is preferable that the constituent fiber that forms the hydrophobic layer 4 and the constituent fiber that forms the hydrophilic layer 5 differ from each other at least in the degrees of hydrophilicity, of the degrees of hydrophilicity, the fineness, the fiber density, and the constituent resin. In particular, with regard to the difference in degrees of hydrophilicity, specifically, the difference is preferably 10 degrees or greater, and more preferably 20 degrees or greater in terms of the difference in contact angle of the constituent fibers as measured in accordance with the method described in "Method for Measuring Contact Angle" above.

In the case where each sweat-absorbent and quick-drying sheet KS is formed of a single sheet of multilayered nonwoven fabric, in order to enable absorption of even small drops of sweat excreted at the onset of sweating, the ratio of the entire area of the fusion-bonded joined regions 8 to the entire area of the sweat-absorbent and quick-drying sheet KS is preferably 15% or less, and more preferably 13% or less, is preferably 6% or greater, and more preferably 8% or greater, and, specifically, is preferably from 6 to 15%, and more preferably from 8 to 13%.

Furthermore, from the same point of view, in the case where each sweat-absorbent and quick-drying sheet KS is formed of a single sheet of multilayered nonwoven fabric, the area of each fusion-bonded joined region 8 is preferably 0.25 mm$^2$ or less, and more preferably 0.2 mm$^2$ or less, is preferably 0.05 mm$^2$ or greater, and more preferably 0.1 mm$^2$ or greater, and, specifically, is preferably from 0.05 to 0.25 mm$^2$, and more preferably from 0.1 to 0.2 mm$^2$.

In the case where each sweat-absorbent and quick-drying sheet KS is formed of a single sheet of multilayered nonwoven fabric, in order to achieve a high ability to absorb sweat that has been absorbed and facilitate diffusion of the sweat that has been absorbed, the basis weight of the entire sweat-absorbent and quick-drying sheet KS is more

preferably 30 g/m$^2$ or less, and more preferably 25 g/m$^2$ or less, is preferably 15 g/m$^2$ or greater, and more preferably 18 g/m$^2$ or greater, and, specifically, is preferably from 15 to 30 g/m$^2$, and more preferably from 18 to 25 g/m$^2$.

Furthermore, from the same point of view, in the case where each sweat-absorbent and quick-drying sheet KS is formed of a single sheet of multilayered nonwoven fabric, the basis weight of the hydrophilic sheet 5 forming the sweat-absorbent and quick-drying sheet KS is preferably 5 g/m$^2$ or greater, and preferably 9 g/m$^2$ or greater, is preferably 15 g/m$^2$ or less, and preferably 13 g/m$^2$ or less, and, specifically, is preferably from 5 to 15 g/m$^2$, and preferably from 9 to 13 g/m$^2$.

In the case where each sweat-absorbent and quick-drying sheet KS is formed of a single sheet of multilayered nonwoven fabric, in order to achieve a high ability to absorb sweat that has been absorbed and facilitate diffusion of the sweat, the inter-fiber distance of the constituent fiber forming the hydrophilic layer 5 is preferably shorter than the inter-fiber distance of the fiber forming the hydrophobic layer 4. The inter-fiber distance of the constituent fiber forming the hydrophilic layer 5 is preferably 45 μm or less, and more preferably 35 μm or less, is preferably 5 μm or greater, and more preferably 10 μm or greater, and, specifically, is preferably from 5 to 45 μm, and more preferably from 10 to 35 μm. The inter-fiber distance is measured using the method described above.

In the case where each sweat-absorbent and quick-drying sheet KS is formed of a single sheet of multilayered nonwoven fabric, in order to achieve a high ability to absorb sweat that has been absorbed and facilitate diffusion of the sweat, the hydrophilic layer 5 of the sweat-absorbent and quick-drying sheet KS is preferably a spunbonded layer or a meltblown layer, and more preferably a meltblown layer.

In the case where each sweat-absorbent and quick-drying sheet KS is formed of a single sheet of multilayered nonwoven fabric, in order to improve the smooth, dry feel, the basis weight of the hydrophobic layer 4 of the sweat-absorbent and quick-drying sheet KS is preferably 5 g/m$^2$ or greater, and preferably 9 g/m$^2$ or greater, is preferably 15 g/m$^2$ or less, and preferably 13 g/m$^2$ or less, and, specifically, is preferably from 5 to 15 g/m$^2$, and preferably from 9 to 13 g/m$^2$.

Furthermore, as shown in FIG. 3, in the outer cover 3 of the diaper 1, the hydrophobic sheet 4 and the hydrophilic sheet 5 are joined and fixed to each other via the plurality of fusion-bonded joined regions 8 through fusion bonding, but the hydrophobic sheet 4 and the hydrophilic sheet 5 may further be fixed to each other via an adhesive.

Furthermore, as shown in FIG. 2, in the outer cover 3 of the diaper 1, the elastic members 71 are arranged between the outer sheet 6 and the inner sheet 3i, but the elastic members 71 may not be provided therebetween. In the case where an elasticized region is formed without elastic members 71 provided therein, a stretchable nonwoven fabric that is stretchable in the lateral direction (Y direction) can be used for an outer cover.

Furthermore, as shown in FIG. 2, the above-described disposable diaper 1 is a pull-on disposable diaper in which the outer cover 3 is in the shape of an hourglass or the like continuously extending from the ventral side region A via the crotch region C to the dorsal side region B, but may be a pull-on disposable diaper in which the outer cover 3 is of a separate type and is divided into separate members composed of a ventral side outer cover, a dorsal side outer cover, and a crotch outer cover.

Furthermore, in the above-described disposable diaper 1, the sweat-absorbent and quick-drying sheets KS are disposed on the skin-facing surface side of the waist elasticized portions G1 and the below-waist lower elasticized portions G2. However, sweat-absorbent and quick-drying sheets may also be disposed in the leg elasticized portions G3. In this case, it is preferable that, in the hydrophilic layers of the sweat-absorbent and quick-drying sheets KS, the orientation direction of the constituent fiber forming the hydrophilic layers matches the stretching and contracting direction of that leg elasticized regions. In each of the leg elasticized portions G3 of the diaper 1, the plurality of leg elastic members 72 arranged in a state of being stretched along the peripheral edge of the leg opening LO contract along the peripheral edge of the leg opening LO. Therefore, it is sufficient that the orientation direction of the constituent fiber forming the hydrophilic layer is substantially parallel to the orientation direction of the leg elastic members 72 (stretching and contracting direction of the leg elasticized region). The wording "substantially parallel" as used herein refers to a state in which the angle formed by the orientation direction of the leg elastic members 72 and the orientation direction of the constituent fiber forming the hydrophilic layer is 0 to 20°.

Furthermore, as shown in FIG. 1, the above-described disposable diaper 1 is a pull-on disposable diaper having the outer cover 3, but the disposable diaper 1 may be an open type disposable diaper. In the case of an open type disposable diaper including a liquid permeable topsheet, a sparingly liquid permeable backsheet, and an absorbent member between these sheets, it is sufficient that a sweat-absorbent and quick-drying sheet KS is disposed on the skin side of at least the dorsal side waist flap FB of the ventral side waist flap FA and the dorsal side waist flap FB extending in the lateral direction Y and positioned outward of the front and rear ends, respectively, in the longitudinal direction X of the absorbent member. Furthermore, a configuration may also be adopted in which the hydrophobic sheet 4 is disposed on the skin-facing surface side of the hydrophilic topsheet, and the topsheet and the hydrophobic sheet 4 are joined and fixed to each other via fusion-bonded joined regions 8 and are together used as a sweat-absorbent and quick-drying sheet. In this case, the topsheet corresponds to the hydrophilic sheet 5.

In addition to a disposable diaper such as the above-described disposable diaper 1, the absorbent article of the present invention may be any sanitary napkin that has an elasticized region.

Examples

Hereinafter, the absorbent article of the present invention will be described in greater detail using examples. However, the scope of the present invention is not limited to the following examples.

Example 1

A single sheet of nonwoven fabric formed by fixing two sheets having a rectangular shape with a length of 350 mm in the MD direction and a length of 50 mm in the CD direction to each other was prepared as a sweat-absorbent and quick-drying sheet. The hydrophobic sheet 4 forming the sweat-absorbent and quick-drying sheet was a spunbonded nonwoven fabric having a basis weight of 12 g/m$^2$. The hydrophilic sheet 5 forming the sweat-absorbent and quick-drying sheet KS was a spunbonded nonwoven fabric having a basis weight of 18 g/m$^2$. The above-described hydrophobic sheet 4 and hydrophilic sheet 5 were laid one on top of the other and joined and fixed to each other via fusion-bonded joined regions 8, as shown in FIG. 3, using a heat embossing apparatus to thereby produce a sweat-absorbent and quick-drying sheet in the form of a nonwoven fabric. The produced sweat-absorbent and quick-drying sheet of Example 1 was used in such a manner that the hydrophobic sheet 4 constituted the skin-facing surface of the sweat-absorbent and quick-drying sheet. The ratio of the entire area of the fusion-bonded joined regions 8 to the entire area of the produced sweat-absorbent and quick-drying sheet of Example 1 was 3.6%, and the area of each fusion-bonded joined region 8 was 0.8 mm$^2$. The fusion-bonded joined regions 8 were dispersedly arranged such that at least one fusion-bonded joined region was partially disposed in an imaginary circle 1R with a radius of 2 mm.

Example 2

A hydrophobic spunbonded nonwoven fabric of 17 g/m$^2$ was used as the hydrophobic sheet 4, and this nonwoven fabric and the same hydrophilic spunbonded nonwoven fabric as that of Example 1 were laid one on top of the other and joined and fixed to each other via fusion-bonded joined regions 8, as shown in FIG. 3, using a heat embossing apparatus to thereby produce a sweat-absorbent and quick-drying sheet KS in the form of a nonwoven fabric. The ratio of the entire area of the fusion-bonded joined regions 8 to the entire area of the produced sweat-absorbent and quick-drying sheet of Example 2 was 12.0%, and the area of each fusion-bonded joined region 8 was 1.3 mm$^2$. The fusion-bonded joined regions 8 were dispersedly arranged such that at least one fusion-bonded joined region was partially disposed within an imaginary circle 1R with a radius of 2 mm.

Example 3

A hydrophobic spunbonded nonwoven fabric of 9 g/m$^2$ was used as the hydrophobic sheet 4, this nonwoven fabric and the same hydrophilic spunbonded nonwoven fabric as that of Example 1 were laid one on top of the other and joined and fixed to each other via fusion-bonded joined regions 8, as shown in FIG. 3, using a heat embossing apparatus to thereby produce a sweat-absorbent and quick-drying sheet in the form of a nonwoven fabric. The ratio of the entire area of the fusion-bonded joined regions 8 to the entire area of the produced sweat-absorbent and quick-drying sheet of Example 3 was 6.0%, and the area of each fusion-bonded joined region 8 was 1.3 mm$^2$. The fusion-bonded joined regions 8 were dispersedly arranged such that not every imaginary circle 1R with a radius of 2 mm partially contained one fusion-bonded joined region, but every imaginary circle 1R with a radius of 3 mm partially contained at least one fusion-bonded joined region.

Example 4

A sweat-absorbent and quick-drying sheet of Example 4 was produced in the same manner as the nonwoven fabric of Example 3 except that a hydrophilic meltblown nonwoven fabric having a basis weight of 20 g/m$^2$ was used as the hydrophilic sheet 5.

Comparative Example 1

A sweat-absorbent and quick-drying sheet was produced in which an air-through nonwoven fabric obtained by blending a hydrophilic fiber in an amount of 75% and a hydrophobic fiber in an amount of 25% and having a basis weight shown in Table 1 below was used as the sheet forming the skin-facing surface, a hydrophilic spunbonded nonwoven fabric having a basis weight shown in Table 1 below was used as the sheet forming the non-skin-facing surface, and the air-through nonwoven fabric and the hydrophilic spunbonded nonwoven fabric were bonded to each other with an adhesive. The blended air-through nonwoven fabric forming the skin-facing surface and the hydrophilic sheet were not joined to each other through fusion-bonding using a heat embossing apparatus and thus had no fusion-bonded joined regions 8.

Comparative Example 2

A sweat-absorbent and quick-drying sheet of Comparative Example 2 was produced in the same manner as the nonwoven fabric of Example 3 except that the hydrophobic sheet 4 was changed to a hydrophilic spunbonded nonwoven fabric having a basis weight of 17 g/m$^2$.

Comparative Example 3

A sweat-absorbent and quick-drying sheet of Comparative Example 3 was produced in the same manner as the sweat-absorbent and quick-drying sheet of Example 1 except that a hydrophobic spunbonded nonwoven fabric having a basis weight of 17 g/m$^2$ was used as the hydrophobic sheet 4, and the hydrophilic sheet 5 was changed to a hydrophobic spunbonded nonwoven fabric having a basis weight of 36 g/m$^2$.

Evaluation of Amount of Remaining Liquid, Liquid Evaporation Time, Difference $\Delta$q-max (49), and Difference $\Delta$q-max (245)

With respect to the sweat-absorbent and quick-drying sheets of Examples 1 to 4 and Comparative Examples 1 to 3, the amount of remaining liquid, the liquid evaporation time, the difference $\Delta$q-max (49), and the difference $\Delta$q-max (245) were measured using the above-described methods. Table 1 below shows the results.

Performance Evaluation

The performance of the sweat-absorbent and quick-drying sheets obtained in Examples 1 to 4 and the sheets obtained in Comparative Examples 1 to 3 was evaluated in the following manner.
Test pieces having dimensions of 5 cm $\times$ 5 cm were cut from the sweat-absorbent and quick-drying sheets obtained in the examples and the comparative examples and used as evaluation samples.
Panelists stayed in a testing room under a constant temperature and humidity environment at a room temperature of 27°C and a relative humidity of 50% for 5 minutes. After that, the evaluation samples were attached to left and right upper back portions of each panelist so that the skin-facing surfaces of the evaluation samples were in contact with the panelist's skin, and a perspiration meter (NS5100 (PMX-002) manufactured by Skinos) was attached to the panelist's skin within 5 cm from the evaluation samples. Each panelist sat on a chair, took a foot bath by soaking both feet up to upper portions of the ankles in hot water at 41 °C, and stayed at rest. After a state in which the amount of perspiration measured on the perspiration meter was greater than 0.1 mg/min/cm$^2$ continued for 25 minutes, the panelist took the feet out of the hot water and stayed at rest for 5 minutes. Then, the samples were removed, and the texture of the skin was evaluated based on the following evaluation criteria.

4 points: No sweat remained on the skin, and the skin was smooth and dry after sweat was absorbed for 5 minutes.

3 points: No sweat remained on the skin, and the skin was not sticky after sweat was absorbed for 5 minutes.

2 points: No sweat remained on the skin, but the skin was sticky after sweat was absorbed for 5 minutes.

1 point: Sweat remained on the skin, and the skin was sticky after sweat was absorbed for 5 minutes.

Five panelists evaluated the samples in the same manner, and the ability to absorb sweat and quickly dry was evaluated based on an average of the evaluation values.
Furthermore, all of the sweat-absorbent and quick-drying sheets obtained in Examples 1 to 4 had a plurality of depressed portions and had fusion-bonded joined regions described above within the respective depressed portions. In addition, in the sweat-absorbent and quick-drying sheet obtained in Examples 1 to 4, the thickness of each of the depressed portions became smaller toward the fusion-bonded joined region in that depressed portion. Moreover, with regard to each of the sweat-absorbent and quick-drying sheets obtained in Examples 1 to 4, when measurement was performed in the above-described manner, it was found that the thickness of the fusion-bonded joined regions was 1/3 or less of the thickness of portions of the sweat-absorbent and quick-drying sheet other than the depressed portions.

Table 1

| | | Unit | Ex. 1 | Ex. 2 | Ex. 3 | Ex. 4 | Com. Ex. 1 | Com. Ex. 2 | Com. Ex. 3 |
|---|---|---|---|---|---|---|---|---|---|
| Skin-facing surface | Type | - | Spunbonded nonwoven fabric | Spunbonded nonwoven fabric | Spunbonded nonwoven fabric | Spunbonded nonwoven fabric | Air-through nonwoven fabric | Spunbonded nonwoven fabric | Spunbonded nonwoven fabric |
| | Hydrophobic/ Hydrophilic | | Hydrophobic | Hydrophobic | Hydrophobic | Hydrophobic | Blend (Hydrophobic/ Hydrophilic) | Hydrophilic | Hydrophobic |
| | Basis weight | - | 12 | 17 | 9 | 9 | 18 | 17 | 17 |
| Non-skin-facing surface | Type | - | Spunbonded nonwoven fabric | Spunbonded nonwoven fabric | Spunbonded nonwoven fabric | Meltblown nonwoven fabric | Spunbonded nonwoven fabric | Spunbonded nonwoven fabric | Spunbonded nonwoven fabric |
| | Hydrophobic/ Hydrophilic | | Hydrophilic | Hydrophilic | Hydrophilic | Hydrophilic | Hydrophilic | Hydrophilic | Hydrophobic |
| | Basis weight | - | 18 | 18 | 18 | 20 | 18 | 18 | 36 |
| Fusion-bonded joined region | Ratio | % | 3.57 | 12 | 6 | 6 | 0 | 6 | 3.57 |
| | Area per single fusion-bonded joined region | $mm^2$ | 0.785 | 1.285 | 1.285 | 1.285 | 0.000 | 1.285 | 0.785 |
| | Radius of imaginary circle (in which fusion-bonded joined region is partially contained) | mm | 2.0 | 1.3 | 3.0 | 3.0 | - | 3.0 | 2.0 |
| Evaluation | Amount of remaining liquid | mg | 1.9 | 1.9 | 1.5 | 1.5 | 32.1 | 12 | 1.5 |
| | Liquid evaporation time | min. | 10.1 | 11.2 | 9.6 | 11.1 | 50.8 | 11.0 | 16.2 |
| | Difference $\Delta$q-max (49) | $kW/m^2$ | 0.08 | 0.06 | 0.10 | 0.07 | 0.23 | 0.3 | 0.08 |
| | Difference $\Delta$q-max (245) | $kW/m^2$ | 0.28 | 0.25 | 0.35 | 0.22 | 0.65 | 0.6 | 0.25 |
| Effect on the skin | | | 3.6 | 3.8 | 3.1 | 3.8 | 1.4 | 2.0 | 2.5 |

According to the results shown in Table 1, the sweat-absorbent and quick-drying sheets of Examples 1 to 4 each exhibited a smaller amount of remaining liquid and a shorter liquid evaporation time compared with the sheets of Comparative Examples 1 to 3. Moreover, the sweat-absorbent and quick-drying sheets of Examples 1 to 4 each exhibited a smaller value of the difference Δq-max (49) compared with the sheets of Comparative Examples 1 to 3. Furthermore, the sweat-absorbent and quick-drying sheets of Examples 1 to 4 each exhibited a smaller value of the difference Δq-max (245) compared with the sheets of Comparative Examples 1 to 3. It was found that, as shown in Table 1, the sweat-absorbent and quick-drying sheets of Examples 1 to 4, which satisfied the above-described requirements, had a higher ability to absorb sweat and quickly dry compared with the sheets of Comparative Examples 1 to 3. Therefore, when a pull-on disposable diaper is used in which the sweat-absorbent and quick-drying sheet of any of Examples 1 to 4 is disposed on the skin-facing surface side of an elasticized region, small drops of sweat excreted at the onset of sweating can be absorbed, and thus, a reduction of skin issues such as eczema, heat rashes, and rashes can be expected.

Industrial Applicability

According to the present invention, both absorption of sweat and quick-drying after absorbing sweat are achieved. Thus, small drops of sweat excreted at the onset of sweating can be absorbed, and skin issues such as eczema, heat rashes, and rashes can be reduced.

**Claims**

1.    An absorbent article comprising an elasticized region,

wherein the elasticized region has, on a skin-facing surface side thereof, a sweat-absorbent and quick-drying sheet (KS) having at least two layers,
the sweat-absorbent and quick-drying sheet (KS) includes a hydrophobic layer (4) forming a skin-facing surface thereof, and a hydrophilic layer (5) disposed adjacent to a non-skin-facing surface of the hydrophobic layer (4), and has a plurality of fusion-bonded joined regions (8) via which the hydrophobic layer (4) and the hydrophilic layer (5) are fixed to each other,
the hydrophobic layer (4) is formed of a hydrophobic fiber and being hydrophobic,
the hydrophilic layer (5) is formed of a hydrophilic fiber and being hydrophilic, and
the sweat-absorbent and quick-drying sheet (KS) is a sheet in which, when 1 μL × 49 drops of a liquid are arranged within an area of 5 cm × 5 cm of the skin-facing surface thereof and absorbed for 60 seconds, an amount of remaining liquid is 10 mg or less, and 50 μL × 1 drop of the liquid placed on the skin-facing surface evaporates within 15 minutes, the amount of remaining liquid being measured as described in paragraphs [0041] of the A1-publication, the liquid evaporation time being measured as described in paragraphs [0044] of the A1-publication,
further comprising an outer cover (3),
wherein the outer cover (3) has an inner sheet (3i), an outer sheet (6) and a hydrophilic sheet (5) separate from the outer sheet (6),
the outer sheet (6) has a plurality of openings (61) passing through the outer sheet (6),
the outer sheet (6) has a folded-back portion (6R) formed by the outer sheet (6) being folded back so as to cover a skin-facing surface side of the hydrophilic sheet (5), and
the hydrophobic layer (4) of the sweat-absorbent and quick-drying sheet (KS) is formed of the folded-back portion (6R) of the outer sheet (6) and the hydrophilic layer (5) of the sweat-absorbent and quick-drying sheet (KS) is formed of the hydrophilic sheet (5) laid one on top of the other,
wherein the openings (61) of the outer sheet (6) are arranged such that at least some of the openings (61) do not overlap the fusion-bonded joined regions (8) of the sweat-absorbent and quick-drying sheet (KS).

2.    The absorbent article according to claim 1, wherein the sweat-absorbent and quick-drying sheet (KS) is a sheet in which a difference Δq-max (49) between a maximum heat transfer rate of the skin-facing surface in a wet state when 1 μL × 49 drops of a liquid have been absorbed within an area of 5 cm × 5 cm of the skin-facing surface for 2 minutes and a maximum heat transfer rate of the skin-facing surface in a humidity-controlled state is 0.15 kW/m$^2$ or less.

3.    The absorbent article according to claim 1 or 2, wherein the sweat-absorbent and quick-drying sheet (KS) is a sheet in which a difference Δq-max (245) between a maximum heat transfer rate of the skin-facing surface in a wet state when 5 μL × 49 drops of a liquid have been absorbed within an area of 7 cm × 7 cm of the skin-facing surface for 2 minutes and a maximum heat transfer rate of the skin-facing surface in a humidity-controlled state is 0.3 kW/m$^2$ or less.

4. The absorbent article according to any one of claims 1 to 3, wherein the fusion-bonded joined regions (8) are dispersedly arranged over the entire hydrophobic layer (4) so that, in a plan view of the sweat-absorbent and quick-drying sheet (KS), at least one fusion-bonded joined region is partially contained in an imaginary circle with a radius of 3 mm.

5. The absorbent article according to any one of claims 1 to 4, wherein, in the hydrophilic layer (5) of the sweat-absorbent and quick-drying sheet (KS), an orientation direction of the constituent fiber forming the hydrophilic layer (5) matches a stretching and contracting direction of the elasticized region.

6. The absorbent article according to any one of claims 1 to 5, wherein the sweat-absorbent and quick-drying sheet (KS) is formed of a single sheet of nonwoven fabric,

   a ratio of an entire area of the fusion-bonded joined regions to an entire area of the sweat-absorbent and quick-drying sheet is 15% or less, and
   an area of each fusion-bonded joined region is 0.25 mm$^2$ or less.

7. The absorbent article according to any one of claims 1 to 6, wherein the sweat-absorbent and quick-drying sheet (KS) has a plurality of through holes passing through the sweat-absorbent and quick-drying sheet (KS).

8. The absorbent article according to any one of claims 1 to 5, wherein the sweat-absorbent and quick-drying sheet (KS) is formed of a plurality of sheets laid one on top of another, and
   a hydrophilic sheet (5) serving as the hydrophilic layer is a spunbonded nonwoven fabric.

9. The absorbent article according to any one of claims 1 to 6, wherein the sweat-absorbent and quick-drying sheet (KS) is formed of a single sheet of multilayered nonwoven fabric, and
   a ratio of an entire area of the fusion-bonded joined regions (8) to an entire area of the sweat-absorbent and quick-drying sheet is from 6 to 15%.

10. The absorbent article according to any one of claims 1 to 6 or 9, wherein the sweat-absorbent and quick-drying sheet (KS) is formed of a single sheet of nonwoven fabric, and
    the hydrophilic layer (5) is a meltblown layer.

11. The absorbent article according to any one of claims 1 to 7 or 8 to 10, wherein the elasticized region has an outer sheet positioned on a non-skin-facing surface side and forming an outer face of the absorbent article, and an inner sheet positioned on a skin-facing surface side of the outer sheet,

    a folded-back portion (6R) of the outer sheet (6) is provided on a skin-facing surface side of the inner sheet, and the sweat-absorbent and quick-drying sheet (KS) is disposed on a side that is closest to the skin-facing surface of the elasticized region.

12. The absorbent article according to any one of claims 1 to 5 or 8, wherein the elasticized region has an outer sheet positioned on a non-skin-facing surface side and forming an outer face of the absorbent article, and an inner sheet positioned on a skin-facing surface side of the outer sheet, and
    a sheet material forming the outer sheet is folded back toward a skin-facing surface of a hydrophilic sheet along a peripheral edge of a waist opening of the absorbent article to form a folded-back portion, and the sweat-absorbent and quick-drying sheet is formed by fixing the folded-back portion serving as a hydrophobic sheet positioned on a side that is closest to the skin-facing surface and the hydrophilic sheet to each other via the fusion-bonded joined regions.

13. The absorbent article according to any one of claims 1 to 5, 8 or 12, wherein
    the sweat-absorbent and quick-drying sheet (KS) has depressed portions depressed from the hydrophobic sheet side toward the hydrophilic sheet side in a thickness direction of the sweat-absorbent and quick-drying sheet (KS), and includes the fusion-bonded joined regions in the respective depressed portions, and each depressed portion has a thickness that becomes smaller toward the fusion-bonded joined region (8).

14. The absorbent article according to any one of claims 1 to 5, 8, 12 or 13, wherein
the sweat-absorbent and quick-drying sheet (KS) has depressed portions depressed from the hydrophobic sheet side toward the hydrophilic sheet side in a thickness direction of the sweat-absorbent and quick-drying sheet, and includes the fusion-bonded joined regions in the respective depressed portions, and each fusion-bonded joined region has a thickness that is 1/3 or less of a thickness of portions of the sweat-absorbent and quick-drying sheet other than the depressed portions.

**Patentansprüche**

1. Saugfähiger Artikel, der eine elastische Region aufweist,

   wobei die elastische Region auf einer der Haut zugewandten Oberflächenseite davon eine Schweiß absorbierende und schnell trocknende Lage (KS) mit wenigstens zwei Schichten aufweist,
   die Schweiß absorbierende und schnell trocknende Lage (KS) eine hydrophobe Schicht (4) aufweist, die eine der Haut zugewandte Oberfläche davon bildet, und eine hydrophile Schicht (5), die angrenzend an eine nicht der Haut zugewandte Oberfläche der hydrophoben Schicht (4) angeordnet ist und eine Vielzahl von schmelzgebunden verbundenen Regionen (8) hat, über die die hydrophobe Schicht (4) und die hydrophile Schicht (5) aneinander befestigt sind,
   die hydrophobe Schicht (4) aus einer hydrophoben Faser ausgebildet ist und hydrophob ist,
   die hydrophile Schicht (5) aus einer hydrophilen Faser ausgebildet ist und hydrophil ist, und
   die Schweiß absorbierende und schnell trocknende Lage (KS) eine Lage ist, in der wenn 1 $\mu$L x 49 Tropfen einer Flüssigkeit in einer Fläche von 5 cm x 5 cm auf der der Haut zugewandten Oberfläche davon angeordnet werden und für 60 Sekunden absorbiert werden, eine restliche Flüssigkeitsmenge 10 mg oder weniger beträgt, und 50 $\mu$L x 1 Tropfen der Flüssigkeit, die auf der der Haut zugewandten Oberfläche aufgetragen werden, innerhalb von 15 Minuten verdunsten, wird die restliche Flüssigkeitsmenge gemessen, wie in Absatz [0041] der A1-Veröffentlichung beschrieben, wobei die Zeit der Flüssigkeitsverdunstung gemessen wird, wie in Absatz [0044] der A1-Veröffentlichung beschrieben,
   weiterhin eine äußere Deckschicht (3) aufweisend,
   wobei die äußere Deckschicht (3) eine innere Lage (3i), eine äußere Lage (6) und eine hydrophile Lage (5) getrennt von der äußeren Lage (6) hat,
   die äußere Lage (6) eine Vielzahl von Öffnungen (61) hat, die durch die äußere Lage (6) hindurchgehen,
   die äußere Lage (6) einen zurückgefalteten Bereich (6R) aufweist, der von der äußeren Lage ausgebildet wird, die zurückgefaltet ist, um so eine der Haut zugewandte Oberflächenseite der hydrophilen Lage (5) abzudecken, und
   die hydrophobe Schicht (4) der Schweiß absorbierenden und schnell trocknenden Lage (KS) aus dem zurückgefalteten Bereich (6R) der äußeren Lage (6) ausgebildet ist und die hydrophile Lage (5) der Schweiß absorbierenden und schnell trocknenden Lage (KS) aus der hydrophilen Lage (5) ausgebildet ist, die aufeinander gelegt sind,
   wobei die Öffnungen (61) der äußeren Lage (6) so angeordnet sind, dass wenigstens einige der Öffnungen (61) die schmelzgebunden verbundenen Regionen (8) der Schweiß absorbierenden und schnell trocknenden Lage (KS) nicht überlappen.

2. Saugfähiger Artikel nach Anspruch 1, wobei die Schweiß absorbierende und schnell trocknende Lage (KS) eine Lage ist, in der eine Differenz $\Delta$q-max (49) zwischen einer maximalen Wärmeübertragungsrate der der Haut zugewandten Oberfläche in einem nassen Zustand, wenn 1 $\mu$L x 49 Tropfen einer Flüssigkeit in einem Gebiet von 5 cm x 5 cm der der Haut zugewandten Oberfläche für 2 Minuten absorbiert wurden, und einer maximalen Wärmeübertragungsrate der der Haut zugewandten Oberfläche in einem feuchtigkeitskontrollierten Zustand 0,15 kW/m$^2$ oder weniger beträgt.

3. Saugfähiger Artikel nach Anspruch 1 oder 2, wobei die Schweiß absorbierende und schnell trocknende Lage (KS) eine Lage ist, in der eine Differenz $\Delta$q-max (245) zwischen einer maximalen Wärmeübertragungsrate der der Haut zugewandten Oberfläche in einem nassen Zustand, wenn 5 $\mu$L x 49 Tropfen einer Flüssigkeit in einem Gebiet von 7 cm x 7 cm der der Haut zugewandten Oberfläche für 2 Minuten absorbiert wurden, und einer maximalen Wärmeübertragungsrate der der Haut zugewandten Oberfläche in einem feuchtigkeitskontrollierten Zustand 0,3 kW/m$^2$ oder weniger beträgt.

4. Saugfähiger Artikel nach einem der Ansprüche 1 bis 3, wobei die schmelzgebunden verbundenen Regionen (8) verteilt über die gesamte hydrophobe Schicht (4) angeordnet sind, sodass in einer Draufsicht der Schweiß absorbierenden und schnell trocknenden Lage (KS) wenigstens eine schmelzgebunden verbundene Region teilweise in einem imaginären Kreis mit einem Radius von 3 mm enthalten ist.

5. Saugfähiger Artikel nach einem der Ansprüche 1 bis 4, wobei in der hydrophilen Schicht (5) der Schweiß absorbierenden und schnell trocknenden Lage (KS) eine Ausrichtungsrichtung der konstituierenden Faser, die die hydrophile Schicht (5) bildet, mit einer Ausdehnungs- und Kontraktionsrichtung der elastischen Region übereinstimmt.

6. Saugfähiger Artikel nach einem der Ansprüche 1 bis 5, wobei die Schweiß absorbierende und schnell trocknende Lage (KS) als eine Lage Vliesstoff ausgebildet ist.

   ein Verhältnis einer Gesamtfläche der schmelzgebunden verbundenen Regionen zu einer Gesamtfläche der Schweiß absorbierenden und schnell trocknenden Lage 15 % oder weniger beträgt, und
   eine Fläche von einem jeden schmelzgebunden verbundenen Gebiet $0,25$ mm$^2$ oder weniger beträgt.

7. Saugfähiger Artikel nach einem der Ansprüche 1 bis 6, wobei die Schweiß absorbierende und schnell trocknende Lage (KS) eine Vielzahl von Durchgangsöffnungen hat, die durch die Schweiß absorbierende und schnell trocknende Lage (KS) hindurchgehen.

8. Saugfähiger Artikel nach einem der Ansprüche 1 bis 5, wobei die Schweiß absorbierende und schnell trocknende Lage (KS) aus einer Vielzahl von aufeinander gelegten Lagen ausgebildet ist, und
   eine hydrophile Lage (5), die als die hydrophile Schicht dient, ein Spinnvlies ist.

9. Saugfähiger Artikel nach einem der Ansprüche 1 bis 6, wobei die Schweiß absorbierende und schnell trocknende Lage (KS) aus einer Lage eines mehrschichtigen Vliesstoffes ausgebildet ist. und
   ein Verhältnis einer Gesamtfläche der schmelzgebunden verbundenen Regionen (8) zu einer Gesamtfläche der Schweiß absorbierenden und schnell trocknenden Lage 6 bis 15 % beträgt.

10. Saugfähiger Artikel nach einem der Ansprüche 1 bis 6 oder 9, wobei die Schweiß absorbierende und schnell trocknende Lage (KS) als eine einzelne Lage Vliesstoff ausgebildet ist, und
    die hydrophile Lage (5) eine schmelzgeblasene Schicht ist.

11. Saugfähiger Artikel nach einem der Ansprüche 1 bis 7 oder 8 bis 10, wobei die elastische Region eine äußere Lage, die an einer nicht der Haut zugewandten Oberflächenseite angeordnet ist und eine Außenfläche des saugfähigen Artikels bildet, und eine innere Lage, die an einer der Haut zugewandten Oberflächenseite der äußeren Lage angeordnet ist, aufweist,

    ein zurückgefalteter Bereich (6R) der äußeren Lage (6) auf einer der Haut zugewandten Oberflächenseite der inneren Lage vorgesehen ist, und
    die Schweiß absorbierende und schnell trocknende Lage (KS) an einer Seite angeordnet ist, die zur hautseitigen Oberfläche der elastischen Region am nächsten ist.

12. Saugfähiger Artikel nach einem der Ansprüche 1 bis 5 oder 8, wobei die elastische Region eine äußere Lage hat, die an einer nicht der Haut zugewandten Oberflächenseite angeordnet ist und eine Außenfläche des saugfähigen Artikels bildet, und eine innere Lage, die an einer der Haut zugewandten Oberflächenseite der äußeren Lage angeordnet ist, und
    ein die äußere Lage bildendes Lagenmaterial zu einer der Haut zugewandten Oberfläche einer hydrophilen Lage entlang einer Umfangskante einer Mittelteilöffnung des saugfähigen Artikels zurückgefaltet ist, um einen zurückgefalteten Bereich zu bilden, und die Schweiß absorbierende und schnell trocknende Lage ausgebildet wird, indem der zurückgefaltete Bereich, der als eine hydrophobe Lage dient, die an einer Seite angeordnet ist, die der der Haut zugewandten Seite am nächsten ist, und die hydrophile Lage aneinander über die schmelzverbunden verbundenen Regionen befestigt werden.

13. Saugfähiger Artikel nach einem der Ansprüche 1 bis 5, 8 oder 12, wobei die Schweiß absorbierende und schnell trocknende Lage (KS) vertiefte Bereiche hat, die von der Seite der hydrophoben Lage in Richtung der Seite der hydrophilen Lage in einer Dickenrichtung der Schweiß absorbierenden und schnell trocknenden Lage (KS) vertieft sind, und die schmelzgebunden verbundenen Regionen in den jeweiligen vertieften Bereichen aufweist, und ein jeder vertiefte Bereich eine Dicke hat, die in Richtung der schmelzgebunden verbundenen Region (8) kleiner wird.

**14.** Saugfähiger Artikel nach einem der Ansprüche 1 bis 5, 8, 12 oder 13, wobei die Schweiß absorbierende und schnell trocknende Lage (KS) vertiefte Bereiche hat, die von der Seite der hydrophoben Lage in Richtung der Seite der hydrophilen Lage in einer Dickenrichtung der Schweiß absorbierenden und schnell trocknende Lage vertieft sind, und schmelzgebunden verbundene Regionen in den jeweiligen vertieften Bereichen aufweist, und eine jede schmelzgebunden verbundene Region eine Dicke hat, die 1/3 oder weniger als eine Dicke von Bereichen der Schweiß absorbierenden und schnell trocknenden Lage, die sich von den vertieften Bereichen unterscheiden, ist.

**Revendications**

**1.** Article absorbant (1) comprenant une région élastique,

dans lequel la région élastique a, sur un côté de la surface tournée vers la peau de celle-ci, une feuille absorbant la sueur et à séchage rapide (KS) ayant au moins deux couches,
la feuille absorbant la sueur et à séchage rapide (KS) inclut une couche hydrophobe (4) formant une surface tournée vers la peau de celle-ci, et une couche hydrophile (5) disposée adjacente à une surface opposée à la peau de la couche hydrophobe (4), et a une pluralité de régions liées par fusion (8) par l'intermédiaire desquelles la couche hydrophobe (4) et la couche hydrophile (5) sont fixées l'une à l'autre,
la couche hydrophobe (4) est formée d'une fibre hydrophobe et étant hydrophobe,
la couche hydrophile (5) est formée d'une fibre hydrophile et étant hydrophile, et
la feuille absorbant la sueur et à séchage rapide (KS) est une feuille dans laquelle, lorsque 1 μl × 49 gouttes d'un liquide sont agencés à l'intérieur d'une zone de 5 cm x 5 cm de la surface tournée vers la peau de celle-ci et absorbés pendant 60 secondes, une quantité de liquide restant est de 10 mg ou moins, et 50 μl × 1 goutte du liquide placé sur la surface tournée vers la peau s'évaporent en 15 minutes, la quantité de liquide restant étant mesurée tel que décrit dans le paragraphe [0041] de la publication AI, la durée d'évaporation du liquide étant mesurée tel que décrit dans le paragraphe [0044] de la publication AI,
comprenant en outre un revêtement externe (3), dans lequel le revêtement externe (3) a une feuille interne (3i), une feuille externe (6) et une feuille hydrophile (5) séparée de la feuille externe (6),
la feuille externe (6) a une pluralité d'ouvertures (61) traversant la feuille externe (6),
la feuille externe (6) a une partie repliée (6R) formée par la feuille externe (6) étant repliée pour couvrir un côté de surface tournée vers la peau de la feuille hydrophile (5), et
la couche hydrophobe (4) de la feuille absorbant la sueur et à séchage rapide (KS) est formée de la partie repliée (6R) de la feuille externe (6) et la couche hydrophile (5) de la feuille absorbant la sueur et à séchage rapide (KS) est formée de la feuille hydrophile (5) posée par-dessus l'autre,
dans lequel les ouvertures (61) de la feuille externe (6) sont agencées de telle manière qu'au moins certaines des ouvertures (61) ne chevauchent pas les régions liées par fusion (8) de la feuille absorbant la sueur et à séchage rapide (KS).

**2.** Article absorbant selon la revendication 1, dans lequel la feuille absorbant la sueur et à séchage rapide (KS) est une feuille dans laquelle une différence Δq-max (49) entre un taux de transfert de chaleur maximal de la surface tournée vers la peau dans un état mouillé lorsque 1 μl × 49 gouttes d'un liquide ont été absorbés dans une zone de 5 cm x 5 cm de la surface tournée vers la peau pendant 2 minutes et un taux de transfert de chaleur maximal de la surface tournée vers la peau dans un état d'humidité régulée est de 0,15 kW/m² ou moins.

**3.** Article absorbant selon la revendication 1 ou 2, dans lequel la feuille absorbant la sueur et à séchage rapide (KS) est une feuille dans laquelle une différence Δq-max (245) entre un taux de transfert de chaleur maximal de la surface tournée vers la peau dans un état mouillé lorsque 5 μl × 49 gouttes d'un liquide ont été absorbés dans une zone de 7 cm x 7 cm de la surface tournée vers la peau pendant 2 minutes et un taux de transfert de chaleur maximal de la surface tournée vers la peau dans un état d'humidité régulée est de 0,3 kW/m² ou moins.

**4.** Article absorbant selon l'une quelconque des revendications 1 à 3, dans lequel les régions liées par fusion (8) sont agencées de manière dispersées sur la totalité de la couche hydrophobe (4) de manière à ce que, dans une vue en plan de la feuille absorbant la sueur et à séchage rapide (KS), au moins une région liée par fusion est partiellement contenue dans un cercle imaginaire avec un rayon de 3 mm.

**5.** Article absorbant selon l'une quelconque des revendications 1 à 4, dans lequel, dans la couche hydrophile (5) de la feuille absorbant la sueur et à séchage rapide (KS), une direction d'orientation de la fibre constitutive formant la couche hydrophile (5) correspond à une direction d'étirement et de contraction de la région élastique.

**6.** Article absorbant selon l'une quelconque des revendications 1 à 5, dans lequel la feuille absorbant la sueur et à séchage rapide (KS) est formée en une seule feuille de tissu non tissé,

un rapport d'une zone totale des régions liées par fusion sur une zone totale de la feuille absorbant la sueur et à séchage rapide est de 15 % ou moins, et
une zone de chaque région liée par fusion est de 0,25 mm$^2$ ou moins.

**7.** Article absorbant selon l'une quelconque des revendications 1 à 6, dans lequel la feuille absorbant la sueur et à séchage rapide (KS) a une pluralité de trous débouchants traversant la feuille absorbant la sueur et à séchage rapide (KS).

**8.** Article absorbant selon l'une quelconque des revendications 1 à 5, dans lequel la feuille absorbant la sueur et à séchage rapide (KS) est formée d'une pluralité de feuilles posées les unes sur les autres, et
une couche hydrophile (5) servant de couche hydrophile est un tissu non tissé filé-lié.

**9.** Article absorbant selon l'une quelconque des revendications 1 à 6, dans lequel la feuille absorbant la sueur et à séchage rapide (KS) est formée d'une feuille unique de tissu non tissé à couches multiples, et
un rapport d'une zone totale des régions liées par fusion (8) sur une zone totale de la feuille absorbant la sueur et à séchage rapide est de 6 à 15 %.

**10.** Article absorbant selon l'une quelconque des revendications 1 à 6 ou 9, dans lequel la feuille absorbant la sueur et à séchage rapide (KS) est formée d'une feuille unique de tissu non tissé, et
la couche hydrophile (5) est une couche obtenue par fusion-soufflage.

**11.** Article absorbant selon l'une quelconque des revendications 1 à 7 ou 8 à 10, dans lequel la région élastique a une feuille externe positionnée sur un côté de surface opposée à la peau et formant une face externe de l'article absorbant, et une feuille interne positionnée sur un côté de surface tournée vers la peau de la feuille externe,

une partie repliée (6R) de la feuille externe (6) est fournie sur un côté de surface tournée vers la peau de la feuille interne, et
la feuille absorbant la sueur et à séchage rapide (KS) est disposée sur un côté qui est le plus proche de la surface tournée vers la peau de la région élastique.

**12.** Article absorbant selon l'une quelconque des revendications 1 à 5 ou 8, dans lequel la région élastique a une feuille externe positionnée sur un côté de surface opposée à la peau et formant une face externe de l'article absorbant, et une feuille interne positionnée sur un côté de surface tournée vers la peau de la feuille externe, et
un matériau de feuille formant la feuille externe est replié en direction d'une surface tournée vers la peau d'une feuille hydrophile le long d'un bord périphérique d'une ouverture de taille de l'article absorbant pour former une partie repliée, et la feuille absorbant la sueur et à séchage rapide est formée par fixation de la partie repliée servant de feuille hydrophobe positionnée sur un côté qui est le plus proche de la surface tournée vers la peau et la feuille hydrophile l'une à l'autre par l'intermédiaire des régions liées par fusion.

**13.** Article absorbant selon l'une quelconque des revendications 1 à 5, 8 ou 12, dans lequel
la feuille absorbant la sueur et à séchage rapide (KS) a des parties renfoncées, renfoncées depuis le côté de feuille hydrophobe en direction du côté de feuille hydrophile dans une direction de l'épaisseur de la feuille absorbant la sueur et à séchage rapide (KS), et inclut les régions liées par fusion dans les parties renfoncées respectives, et chaque partie renfoncée a une épaisseur qui diminue en direction de la région liée par fusion (8).

**14.** Article absorbant selon l'une quelconque des revendications 1 à 5, 8, 12 ou 13, dans lequel
la feuille absorbant la sueur et à séchage rapide (KS) a des parties renfoncées, renfoncées depuis le côté de feuille hydrophobe en direction du côté de feuille hydrophile dans une direction d'épaisseur de la feuille absorbant la sueur et à séchage rapide, et inclut les régions liées par fusion dans les parties renfoncées respectives, et chaque région liée par fusion a une épaisseur qui est 1/3 ou moins d'une épaisseur de parties de la feuille absorbant la sueur et à séchage rapide autres que les parties renfoncées.

[Fig. 1]

[Fig. 2]

[Fig. 3]

[Fig. 4]

[Fig. 5]

[Fig. 6]

[Fig. 7]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004298467 A
- JP 2002020957 A
- JP H931823 A
- JP 2008229246 A